(19) Europäisches Patentamt
European Patent Office
Office européen des brevets

(11) EP 3 220 818 B1

(12) **EUROPÄISCHE PATENTSCHRIFT**

(45) Veröffentlichungstag und Bekanntmachung des Hinweises auf die Patenterteilung:
**21.08.2019 Patentblatt 2019/34**

(51) Int Cl.:
**A61B 5/083** (2006.01) **G01N 33/497** (2006.01)
**G16Z 99/00** (2019.01)

(21) Anmeldenummer: **15777650.1**

(22) Anmeldetag: **01.10.2015**

(86) Internationale Anmeldenummer:
**PCT/EP2015/072693**

(87) Internationale Veröffentlichungsnummer:
**WO 2016/050911 (07.04.2016 Gazette 2016/14)**

(54) **13C-ATEMGAS-TEST ZUR ÜBERPRÜFUNG DER MAGEN-/DARM-FUNKTION UND/ODER STOFFWECHSELFUNKTIONEN UND VORRICHTUNG HIERFÜR**

13C BREATH TEST FOR CHECKING THE GASTROINTESTINAL FUNCTION AND/OR METABOLIC FUNCTIONS AND DEVICE THEREFOR

TEST RESPIRATOIRE AU CARBONE 13C PERMETTANT DE VÉRIFIER LA FONCTION GASTRO-INTESTINALE ET/OU LES FONCTIONS MÉTABOLIQUES ET DISPOSITIF AFFÉRENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.10.2014 DE 102014014671**

(43) Veröffentlichungstag der Anmeldung:
**27.09.2017 Patentblatt 2017/39**

(73) Patentinhaber: **Fischer Analysen Instrumente GmbH**
**04347 Leipzig (DE)**

(72) Erfinder:
• **KUHLMANN, Bernd**
**04824 Beucha (DE)**
• **VOGT, Josef**
**89231 Neu-Ulm (DE)**

• **FABINSKI, Walter**
**65830 Kriftel (DE)**
• **SCHAICH, Martin**
**04105 Leipzig (DE)**

(74) Vertreter: **Höfer, Friederike**
**Dreiköniggasse 10**
**89073 Ulm (DE)**

(56) Entgegenhaltungen:
**WO-A2-03/017818    DE-A1-102009 039 543**
**US-A1- 2011 313 677**

• **SKURIDA G I: "Representation of the characteristic equation of the unbranched enzymatic reaction with an arbitrary number of stages in the form of a polynomial.", BIOPHYSICS, Bd. 25, Mai 1980 (1980-05), Seiten 443-446, XP008178133,**

EP 3 220 818 B1

**Beschreibung**

Die Erfindung betrifft einen $^{13}$C-Atemgas-Test zur Überprüfung der Magen-/Darm-Funktion und/oder Stoffwechselfunktionen und eine Vorrichtung hierfür.

## HINTERGRUND DES STANDES DER TECHNIK

Derzeit wird die Funktionalität des Magen-/Darm-Traktes über allgemeine medizinische Kriterien, wie Darmgeräusche, Spannung der Bauchdecke und Stuhlgang, untersucht oder über die Bestimmung des Volumens des Mageninhalts, das nach einer bestimmten Zeit nach Verabreichung vorgefunden wird (auch bekannt als "GRV", gastric residual volume). Hierfür wird einem Patienten über eine Magensonde, die über die Nase eingeführt wird, eine Nährstofflösung zugeführt. Danach wird die Magensonde mit einem leeren Beutel verbunden, in den über das Prinzip der kommunizierenden Röhren der Mageninhalt gesaugt wird. Je größer das zurückfließende Volumen ist, umso schlechter ist die aktuelle Magenentleerung. Dieser einfache Test liefert jedoch nur eine grobe Abschätzung, da das Magenvolumen eine Mischfunktion aus Entleerung, Sekretion der Magensäfte und Rückfluss des Verdauungsbreis aus dem Darm in den Magen ist. Die Menge der Nährstoffe, die tatsächlich weitergegeben wird, wird nicht korrekt erfasst. Zudem ist das Verfahren invasiv und für den Patienten sehr unangenehm. Die Ergebnisse dieses Verfahrens sind zudem sehr umstritten. Darüber hinaus kann über Kontrastmittel im Röntgen- oder MRT-Verfahren die Magenentleerung erfasst werden. Dies ist jedoch als Routine-Einsatz sehr kostspielig, zeitaufwändig und für Intensiv-Patienten, die möglichst wenig bewegt werden sollen, nicht zweckmäßig. Auch mit Hilfe der Endoskopie besteht die Möglichkeit, die Magen-/Darm-Funktion abzuschätzen. Jedoch ist dies arbeitsintensiv, da dies nicht vom Pflegepersonal allein durchzuführen ist, sondern regelmäßig auch ein Arzt beteiligt sein muss. Dies ist zeitaufwändig und zudem für den Patienten mit Risiko verbunden, da eine lokale Anästhesie durchgeführt werden muss. Zudem kann während einer Endoskopie ein Würgen und Erbrechen auftreten, wobei Mageninhalt in die Lunge geraten kann und die Gefahr einer Lungenentzündung besteht. Daher ist dieses invasive Verfahren mit erheblichen Nachteilen für den Patienten verbunden. Ein zuverlässiger, kostengünstiger und nicht-invasiver Routinetest als Grundlage für eine Diagnose bei Magenentleerungsproblemen ist daher bislang nicht bekannt.

Zudem haben Kranke, insbesondere kritisch Kranke, wie Intensiv-Patienten, häufig eine gestörte Magen-/Darm-Funktion, die auf Störungen der Magen-/Darm-Motorik beruht und/oder hormonell bedingt sein kann. Diese Störungen können zu einer Nahrungsmittel-Unverträglichkeit führen und sind mit Permeabilitäts-Störungen verbunden, wobei Bakterien vom Darm in die Blutzirkulation gelangen können, was zu heftigen Entzündungsreaktionen führt. Eine möglichst frühe enterale Ernährung kann diese Symptomatik abmildern. Eine Umstellung auf enterale Ernährung kann durch Medikamente, die die Magenentleerung beschleunigen, (sog. 'prokinetic drugs') können diese Symptomatik abmildern.

Eine Therapiemöglichkeit hierfür ist die künstliche Ernährung. Hierbei besteht die Möglichkeit einer frühzeitigen enteralen Ernährung, d.h. eine Ernährung über den Magen-/Darm-Trakt mit Hilfe einer Magensonde. Eine enterale Ernährung kann eine Erholung des Magen-/Darm-Systems beschleunigen, ist jedoch mit Komplikationen verbunden. Werden die Nährstoffe nicht über den Magen-/Darm-Trakt, sondern durch Infusion direkt in die Blutbahn verabreicht (beispielsweise bei Darmerkrankungen), spricht man von parenteraler Ernährung, die im Vergleich zur enteralen Ernährung mit noch erheblich höheren Komplikationsrisiken verbunden ist. Ob eine enterale oder parenterale Ernährung notwendig ist, stellt für den behandelnden Arzt daher eine kritische Entscheidung dar: Eine zu früh eingesetzte enterale Ernährung birgt das Risiko, dass die Ernährung wegen Unverträglichkeit, wie Durchfall oder Erbrechen, zeitweise unterbrochen oder zumindest reduziert werden muss, so dass der erhöhte Nährstoffbedarf von kritisch kranken Patienten nicht mehr gedeckt werden kann. Eine möglichst schnelle Umstellung auf enterale Ernährung ist daher angestrebt, wobei gleichzeitig Patienten ausgenommen werden müssen, bei denen dies nicht möglich ist. Es wäre daher ein Test hilfreich, mit dem ein Arzt zuverlässige Informationen über die Funktion des Magen-/Darm-Systems erhalten könnte.

Beispielsweise offenbart die WO 03/017818 A2 ein Verfahren zum Bestimmen mindestens eines gastrointestinalen Zustands bei einem Patienten, umfassend die Schritte: Durchführen eines ersten Atemtests, der aus einer Gruppe von Atemtests ausgewählt wurde, wobei jeder Atemtest der Gruppe gastrointestinale Informationen bezüglich des Patienten bereitstellt; Durchführen mindestens eines zweiten Atemtests, der aus der Gruppe von Atemtests gemäß dem Ergebnis mindestens des ersten Atemtests ausgewählt wurde und Bestimmen eines gastrointestinalen Zustands des Patienten aus dem Ergebnis von mindestens einem der Atemtests.

In der klinischen Medizin sind $^{13}$C-Atemgas-Tests zur Beurteilung von einer Reihe an Erkrankungen bekannt geworden. Ein informativer Übersichtsartikel zu den verschiedenen Möglichkeiten, die ein $^{13}$C-Atemgas-Test bietet, sowie die eingesetzten Geräte, mit denen die entsprechenden Messungen durchgeführt werden, geht beispielsweise hervor aus Modak A., Stable isotope breath tests in clinical medicine: a review, J. Breath Res. 2007; 1:014003. Beispielsweise ist ein $^{13}$C-Harnstoff-Atemtest für die Feststellung der H. Pylori-Infektion bekannt und wurde auch von der FDA zugelassen.

Typische Messgröße bei diesen Tests ist der Parameter $\delta^{13}$C. Diese geht von einem messbaren Probenver-

hältnis, etwa $^{13}C/^{12}C_{Probe}$ aus, und wird über folgende Formel berechnet:

$$\delta^{13}C = (\ (^{13}C/^{12}C_{Probe} - {}^{13}C/^{12}C_{Standard})\ /\ (^{13}C/^{12}C_{Standard})\ )\ \text{x } 1000‰$$

Hierzu wird beispielsweise verwiesen auf D.A. Schoeller et al. "13C abundances of nutrients and the effect of variations in 13C isotopic abundances of test meals formulated for 13CO2 breath tests", The American Journal of Clinical Nutrition, 33, 1980, S. 2375-2385 und B. Braden et al., "13C-breath tests: Current state of the art and future directions", Digestive and Liver Disease, 39, 2007, S. 795-805.

So sind beispielsweise $^{13}$C-Atemgas-Tests bei kranken Patienten mit schweren Leber-funktionsstörungen aus der US 2011/0313677 A1 bekannt geworden. Die US-Patentanmeldung bezieht sich auf einen $^{13}$C-Atemgas-Leberfunktionstest bei Patienten mit schweren Leber-Funktionsstörungen, wobei die Testsubstanz intravenös verabreicht wird. Hierbei hängt die $^{13}CO_2$-Freisetzung von der "Anflutung" der Testsubstanz ins Innere der Leberzellen und von der Geschwindigkeit, mit der die Substanz dort verarbeitet wird, ab. Die Anflutung wird über den Anstieg in $^{13}CO_2$-Freisetzungskurven erfasst und gilt als ein Charakteristikum für die Leberfunktion, da dieses das Funktionie-ren der Mikrozirkulation in der Leber erfasst. Jedoch kann dieses Verfahren nicht auf die Magen-/Darm-Entleerung übertragen werden.

Aus dem Stand der Technik ist es auch bekannt, dass die Magenentleerung über einen $^{13}$C-Atemgas-Test bei gesunden Personen ermittelt werden kann (Klein P.D., Clinical applications of 13CO2 measurements, Federation proceedings, 1982; 41(10): 2698-2701 und Braden B., Methods and functions: Breath tests, Best practice & research clinical gastroenterology, 2009; 23(3): 337-352).

Weiterhin werden in der WO 2011/026613 A2 ein Verfahren und eine Einrichtung zur Aufzeichnung und Auswertung von Stoffwechselvorgängen, insbesondere zur Ermittlung der Magenentleerzeit oder der Interaktion bei der Polymedikamentation mit Hilfe einer isotopenspezifischen Analyse, beschrieben. Um hierbei zu erreichen, dass eine zeitnahe Analyse durchgeführt werden kann, und die Problematik der offline-Entnahme überwunden wird, um schneller erforderliche Maßnahmen einleiten zu können, wird vorgeschlagen, dass der $^{13}$C-Atemgaswert des Probanden mittels der NDIR-Spektroskopie (Nicht-Dispersive-Infrarot-Spektroskopie) kontinuierlich, d.h. online, mit einer Periode ermittelt und gespeichert wird, die deutlich kürzer ist als die biologische Halbwertzeit von Veränderungen im Stoffwechsel oder Konzentrationen in den Pools.

Bei kranken Patienten funktionieren die oben beschriebenen bekannten Tests jedoch nicht.

Der $^{13}$C-Atemgas-Test beruht im Wesentlichen auf dem zeitlichen Profil der $^{13}CO_2$-Anreicherung nach Verabreichung einer $^{13}$C-markierten Testverbindung. Hierbei kann man aus dem Anstieg zu einem Maximum, gefolgt von einem exponentiellen "Abklingen" der Anreicherung, den Transport der (zu metabolisierenden) $^{13}$C-markierten Testverbindung vom Magen in stoffwechselaktive Organe ableiten.

Häufig werden bei den bekannten $^{13}$C-Atemgas-Tests jedoch nur charakteristische Größen erfasst, wie der Zeitpunkt, bei dem die Hälfte des $^{13}CO_2$ freigesetzt wird, oder der Zeitpunkt, bei dem die maximale Freisetzung erreicht wird. Eine korrekte Erfassung benötigt jedoch relativ dicht gesetzte Messpunkte. Die Ergebnisse der Tests aus dem Stand der Technik sind daher regelmäßig schwer oder gar nicht mehr interpretierbar.

Vier verschiedene Prozesse beeinflussen die Freisetzung von $^{13}CO_2$ während eines oralen Atemgas-Tests. Dies sind:

    1. Magenentleerung;
    2. Absorption über den Darm;
    3. Verteilung der Testverbindung über die Blutzirkulation; und
    4. Umbau der Testverbindung zu $^{13}CO_2$ und nachfolgendes Abatmen des Produkts.

Es gibt jedoch nur eine Beobachtungsgröße für die 4 oben genannten unbekannten Prozesse, nämlich die $CO_2$-Konzentration in der Atemluft. Deswegen werden Annahmen nötig, wie etwa, dass Schritt 3) so schnell abläuft, dass er den zeitlichen Verlauf der Freisetzung nicht beeinflusst. Dies trifft jedoch nicht immer zu. Ein Atemgas-Test zielt in der Regel darauf ab, einen der oben genannten Prozesse, speziell 1, 2 oder 4 zu erfassen. Dies ist jedoch nicht hinreichend, um zuverlässige Ergebnisse zu erhalten. Der kombinierte Einfluss von Magenentleerung und von anschließender Verstoffwechselung kann nicht ohne weiteres getrennt werden, so dass Auswerte-Verfahren nötig werden, welche die in den Testdaten enthaltene Informationsmenge voll ausschöpfen.

Ein weiteres Problem, das bislang bekannte Atemgas-Tests nicht lösen können, ist die Auswertung von irregulären Freisetzungskurven: Bei funktionierender Magenentleerung und Verstoffwechselung, d.h. bei einer gesunden Person, erhält man eine Freisetzungskurve mit nur einem Maximum, deren Auswertung einfach gehalten werden kann, so dass direkt nach der letzten Messung des Tests die entsprechenden Ergebnisse erhalten werden. Kranke Patienten, Diabetiker oder Patienten, die Medikamente einnehmen, wie beispielsweise Beruhigungsmittel, oder die unter Cytokine-Belastung stehen, haben jedoch eine irreguläre, d.h. mehrphasige Magenentleerung. Das bekannte Verfahren ist daher auf kranke Patienten nicht anwendbar, da dieses voraussetzt, dass die $^{13}CO_2$-Freisetzungskurve eine glatte Funktion mit nur einem ausgeprägten Maximalwert (Peak) ist.

Dies wird beispielsweise in der DE 10 2009 039 543 A1 bestätigt. Diese bezieht sich gemäß Anspruch 1 auf ein Verfahren zur Aufzeichnung und Auswertung von Stoffwechselvorgängen, insbesondere zur Ermittlung der Magenentleerzeit oder der Interaktion bei der Polymedikamentation mit Hilfe der isotopenspezifischen Analyse, bei welchem die Magenentleerzeiten mittels des Quotienten der $^{13}CO_2$- und $^{12}CO_2$-Konzentration in der Probanden-Expirationsluft ermittelt wird, wobei der $^{13}C$-Atemgaswert des Probanden mittels NDIR-Spektroskopie (Nicht-Dispersive-Infrarot-Spektroskopie) kontinuierlich, d. h. online, mit einer Periode ermittelt und gespeichert wird, die deutlich kürzer ist als die biologische Halbwertzeit von Veränderungen im Stoffwechsel oder Konzentrationen in den Pools. In diesem Dokument wird explizit angegeben, dass der Test für schwerkranke Patienten entweder generell nicht anwendbar ist oder unzuverlässige Ergebnisse liefert. Das Auswerte-Verfahren versagt vollständig, wenn die Magenentleerung zeitweise blockiert und diskontinuierlich wird. Auswerteverfahren für diesen Fall sind nicht etabliert. Es gibt bislang auch keine Ansätze aus dem Stand der Technik, Störungen im Darmbereich und Störungen in der Magen-Entleerung getrennt zu erfassen.

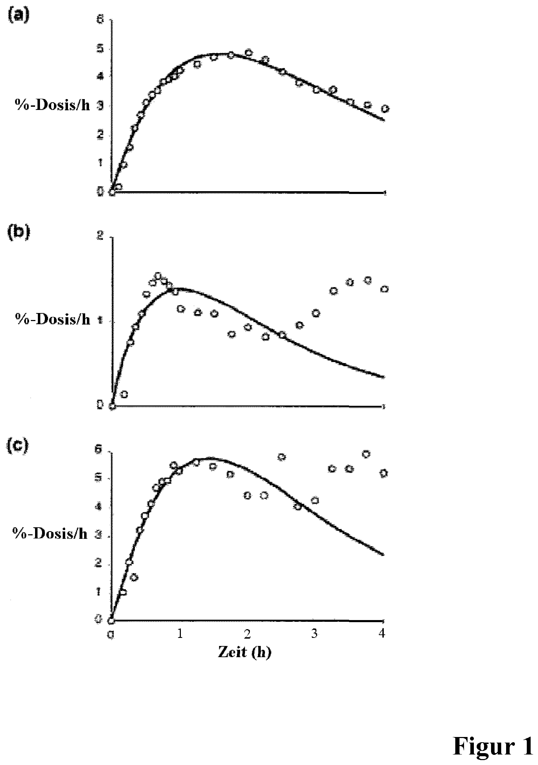

Zur Veranschaulichung der obigen Ausführungen sind in Figur 1 drei Freisetzungskurven abgebildet: die oberste Freisetzungskurve (a) ist von einem gesunden Patienten; es wird wie erwartetet eine Freisetzungskurve mit nur einem Maximum erhalten. Die Freisetzungskurven (b) und (c) hingegen sind von kranken Patienten oder Patienten, die Medikamente einnehmen, so dass die Magenentleerung derart gestört ist, dass eine Kurve mit mehreren Peaks (s. Figur 1, Patient (b)) oder eine völlig irreguläre Freisetzungskurve (s. Figur 1, Patient (c)) erhalten wird. Die Kurven für die Patienten (b) und (c) sind nicht auswertbar, die Freisetzung der Abbauprodukte über die Atmung kann so nicht ermittelt werden. Der Einsatz eines bekannten $^{13}C$-Atemgas-Tests ist damit beschränkt auf Patienten, bei denen eine Auswertung möglich ist, d.h. auf gesunde Patienten.

Eine Möglichkeit, Studien, bei denen irreguläre Freisetzungskurven auftreten, dennoch auszuwerten, besteht darin, bestimmte Datensätze von der Untersuchung auszuschließen. Dies führt jedoch dazu, dass der Verwender bei Untersuchungen am Patienten, beispielsweise im Rahmen von klinischen Studien, in die Auswertung subjektiv eingreift, so dass keine objektive und reproduzierbare Auswertung unabhängig vom Verwender mehr möglich ist. Weiterhin müssen bei standardisierten Vorgaben Einschränkungen der Auswertequalität in Kauf genommen werden. Daher waren bisher die Voraussetzungen für die zuverlässige Durchführung und Anwendung eines $^{13}C$-Atemgas-Tests bei Kranken nicht gegeben.

Ein prinzipielles Problem bei $^{13}C$-Atemgas-Tests ist weiterhin, dass die Freisetzungskurven in der Atemluft immer eine Mischfunktion aus Magen-/Darm-Passage und anschließender Oxidation darstellen. Bei kranken und insbesondere kritisch kranken Personen ist jedoch der Stoffwechsel im Allgemeinen derart gesteigert, dass bei Verwendung, beispielsweise von Acetat als Testverbindung, im $^{13}C$-Atemgas-Test die Anreicherung von $^{13}C$-Test-Acetat durch körpereigene Acetat-Produktion verdünnt wird und gleichzeitig ein erhöhter Anteil des verdünnten $^{13}C$-Acetats für andere Prozesse als die Oxidation verwendet wird, wodurch der Anteil der Testverbindung, der oxidiert wird, erheblich vermindert wird. Ein niedrigerer $^{13}CO_2$-Freisetzungswert kann daher eine reduzierte Magen-/Darm-Passage, eine reduzierte Oxidation der Testverbindung oder eine erhöhte Verdünnung der Anreicherung der Testverbindung zu einem bestimmten Zeitpunkt bedeuten. Die Ergebnisse sind nicht eindeutig und schwer zu interpretieren.

Der Anteil, der oxidiert wird, kann auch aus der kumulativ freigesetzten $^{13}CO_2$-Menge abgeschätzt werden, dies setzt jedoch Messungen voraus, die solange durchgeführt werden, bis nahezu alles $^{13}CO_2$ abgeatmet ist. $^{13}CO_2$ wird jedoch nicht direkt nach der Oxidation abgeatmet, sondern verteilt sich über den Körper, so dass eine vollständige Freisetzung erst Stunden nach der abgeschlossenen Magen-/Darm-Passage zu erwarten ist. Die Erfassung der vollständigen Freisetzung ist somit langwierig und sollte, wenn möglich, vermieden werden.

Um daher zu zuverlässigen Werten zu kommen, müssten eigentlich die bei Kranken parallel auftretenden Störungen der Magenentleerung und des Stoffwechsels getrennt bestimmt werden. Eine getrennte Bestimmung setzt jedoch eine aufwändige mathematisch gestützte Analyse der Messdaten voraus, mit Interaktionen von entsprechend geschultem Personal. Damit wird ein standardisierter "vor Ort"-Test als Unterstützung für die Diagnose, der vom Pflegepersonal durchgeführt werden kann, unmöglich. Mit der Erfassung zusätzlicher Parameter wird die Auswertung immer komplexer, die Einfachheit eines Atemgas-Tests geht verloren und damit die Anwendbarkeit als einfaches Hilfsmittel für eine spätere Diagnose. Als Faustregel gilt, dass je kränker der Patient ist, eine Auswertung der Testdaten um so schwieriger und aufwändiger wird. Eine Überlagerung der Magen-/Darm-Passage mit Stoffwechsel-Prozessen, die alle aus unterschiedlichen Gründen gestört sein können, und gleichzeitig die verzögerte Abgabe über die Lunge, führt dazu, dass ein $^{13}C$-Atemgas-Test bei kranken Patienten, insbesondere Intensiv-Patienten, bislang nicht herangezogen werden konnte. Gerade bei diesen Patienten wäre jedoch ein Test auf noch funktionierende Magen-/Darm-Tätigkeit sinnvoll, da beispielsweise nur bei funktionierendem Magen-/Darm-System eine Ernährung über eine Magensonde möglich ist.

Weiterhin wird in der DE 10 2011 007 310 A1 ein Verfahren zur Bestimmung der metabolischen Leistung mindestens eines Enzyms beschrieben, wobei verschiedene $^{13}C$-markierter Substrate verwendet werden. Das Verfahren umfasst die Schritte der zeitaufgelösten Bestimmung der Konzentration eines Produktes in der Ausatemluft eines Individuums, wobei das Produkt durch eine Metabolisierung eines dem Individuum zuvor verabreichten Substrates durch mindestens ein Enzym des Individuums erzeugt wurde und wobei die Produktkonzentration zumindest bis zum Erreichen der maximalen Produktkonzentration in der Ausatemluft des Individuums bestimmt wird, der Anpassung einer Modellfunktion an Messwerte der Produktkonzentration, die durch die zeitaufgelöste Bestimmung der Produktkonzentration zwischen einem Anfangszeitpunkt und einem Endzeitpunkt erhalten wurden, und der Bestimmung der metabolischen Leistung des Enzyms anhand von Parametern der Modellfunktion, welche die Modellfunktion spezifizieren. Das Verfahren zeichnet sich dadurch aus, dass die Bestimmung der metabolischen Leistung des Enzyms anhand mindestens zweier Parameter der Modellfunktion erfolgt, mit der Maßgabe, dass als Parameter nicht gleichzeitig der Maximalwert der Modellfunktion und die Zeitkonstante der Modellfunktion gewählt werden, sofern die Modellfunktion eine monoexponentielle Funktion ist, und der weiteren Maßgabe, dass der Anfangszeitpunkt und/oder der Endzeitpunkt nicht als Parameter gewählt werden. Hierbei wird die Substanz direkt ins Blut injiziert, eine orale Verabreichung wird als ungeeignet angesehen. Jedoch ist das Risiko einer Injektion für den Patienten deutlich größer als bei einer oralen Gabe. Das Verfahren dient im Wesentlichen zur Überprüfung der Leberfunktion im Hinblick auf eine Lebertransplantation und hat daher eine ganz andere Zielsetzung als das erfindungsgemäße Verfahren. Dieses Verfahren kann daher nur bei Spezialfällen Anwendung finden und soll nicht als Routinediagnostikum dienen.

Schließlich wird noch auf Skurida G. I.: "Representation of the characteristic equation of the unbranched enzymatic reaction with an arbitrary number of stages in the form of a polynomial", Biophysics, Bd. 25, 1980, S. 443-446 verwiesen, worin eine Regel vorgeschlagen wird, die es ermöglicht die charakteristische Gleichung eines Satzes von Differentialgleichungen, die ein Modell für den Verlauf einer enzymatischen Reaktion in Form eines Polynoms mit Koeffizienten, die algebraische Funktionen der kinetischen Konstanten und der Konzentrationen der Substrate und Produkte sind, leichter darzustellen.

Erfindungsgemäß liegt daher die Aufgabe zugrunde, die Nachteile des Standes der Technik zu überwinden. Insbesondere soll ein Test zur Verfügung gestellt werden, der es ermöglicht auch bei Kranken und Intensivpatienten festzustellen, ob das Magen-/Darm-System funktioniert und eine Magenentleerung erfolgt. Weiterhin soll es auch möglich sein, zusätzlich zur Magen-/Darm-Passage Informationen über den Stoffwechsel zu erhalten, wobei mit einer parallelen, getrennten Bestimmung von Magen-Darm-Passage und Stoffwechsel die Aussagekraft der Tests verbessert werden kann. Insbesondere soll hierbei auf bestimmte Stoffwechselparameter abgezielt werden, wie die Gallen- und Leberfunktion. Es soll ein zuverlässiger, kostengünstig durchführbarer Test zur Verfügung gestellt werden, der möglichst wenig oder nicht invasiv ist, routinemäßig in einfacher Weise durchgeführt werden kann und zuverlässige Ergebnisse bereitstellt. Weiterhin soll eine Vorrichtung zur Durchführung des Verfahrens bereitgestellt werden.

## BESCHREIBUNG DER ERFINDUNG

Die oben geschilderte Aufgabe wird gelöst durch einen $^{13}C$-Atemgas-Test zur Überprüfung der Magen-/Darm-Funktion und/oder der Stoffwechselfunktionen bei einem Patienten, umfassend die nachfolgenden Schritte:

- Verabreichen einer mit $^{13}C$-markierten Verbindung an einen Patienten, durch den die $^{13}C$-markierte Verbindung zu einem $^{13}C$-markierten Metabolisierungsprodukt umgewandelt wird, wobei das $^{13}C$-markierte Metabolisierungsprodukt $^{13}CO_2$ darstellt;
- Bestimmen der Änderung der relativen Konzentrationen des $^{13}CO_2$ in der Atemluft des Patienten über ein vorbestimmtes Zeitintervall in Form von gemessenen Anreicherungsdaten; und
- Auswerten der gemessenen Anreicherungsdaten anhand einer mathematischen Abschätzung der $^{13}CO_2$-Freisetzungskurve, die aufweist:

  - Formulieren von Differentialgleichungen zur Erfassung der Kinetik der $^{13}C$-markierten Verbindung im Körper des Patienten anhand bekannter kinetischer Koeffizienten;
  - Erstellen linearer Matrixgleichungen aus den Differentialgleichungen unter Berücksichtigung des zeitlichen Verlaufs des Transfers der $^{13}C$-markierten Testverbindung in die zentrale Zirkulation des Körpers des Patienten anhand einer Transfer-Funktion R(t) in Form eines Polynoms unter Erhalt einer Gleichung, die eine Beziehung zwischen Polynomkoeffizienten für die Transfer-Funktion R(t) und den gemessenen Anreicherungsdaten herstellt (siehe Gleichung (12));
  - Regularisieren der erhaltenen Gleichung durch Kontrolle der Biegsamkeit der Transfer-Funktion R(t) über den Regularisierungskoeffizient $\alpha$ unter Beschränkung der Größe der Polynomkoeffizienten (d.h. die Summe der quadrierten Polynomkoeffizienten wird minimiert), wobei je kleiner die Beschränkung ist, um so größer die Biegsamkeit ist, unter Erhalt einer regularisierten Gleichung (siehe Gleichung (13));
  - Umwandeln der regularisierten Gleichung in eine Wahrscheinlichkeit (siehe Gleichung (15)), dass ein bestimmter Datensatz in Abhängigkeit vom Regularisierungskoeffizienten a, den kinetischen Koeffizienten, einem Stoffwechselparameter $\gamma$ und den Polynomkoeffizienten gemessen wird;
  - Marginalisieren der Wahrscheinlichkeit, so dass nur eine Wahrscheinlichkeit berechnet wird, die vom Regula-

risierungskoeffizienten $\alpha$ und vom Stoffwechselparameter $\gamma$ abhängt, wobei die Polynomkoeffizienten vom Regularisierungskoeffizienten $\alpha$ und vom Stoffwechselparameter $\gamma$ abhängen (siehe Gleichung (14)); und

- automatische Optimierung der Wahrscheinlichkeit unter Variieren des Regularisierungskoeffizienten $\alpha$ und des Stoffwechselparameters $\gamma$ unter Erhalt der wahrscheinlichsten Werte für $\alpha$ und $\gamma$ und damit auch der Polynomkoeffizienten (siehe Gleichung (14));
und

- Rückrechnung der Magen-/Darm-Entleerung R(t) aus den erhaltenen Polynomkoeffizienten (siehe Gleichung (9)) und Bestimmung der Verstoffwechselung der $^{13}C$-markierten Verbindung zu $^{13}CO_2$ aus dem Stoffwechselparameter $\gamma$ (siehe Gleichung (3)).

Gegenstand der Erfindung ist auch eine Vorrichtung zur Durchführung des Verfahrens, umfassend

- eine Messvorrichtung zur Bestimmung der Änderung der relativen Konzentrationen des $^{13}C$-markierten Metabolisierungsprodukts in Form von $^{13}CO_2$ in der Atemluft des Patienten über ein vorbestimmtes Zeitintervall unter Erhalt von Messwerten und
- eine Auswertevorrichtung, die derart eingerichtet ist, dass sie die Anreicherungsdaten gemäß der vorliegenden Erfindung auswertet.

Die Offenbarung betrifft auch die Verwendung einer Vorrichtung zur Durchführung des $^{13}C$-Atemgas-Tests gemäß der Erfindung.

Das Verfahren beruht daher auf einer isotopenspezifischen Atemgasanalyse von $^{13}CO_2$- und $^{12}CO_2$-Konzentrationen nach Verabreichung eines geeignet gelabelten Substrates, das auch als Tracer bezeichnet wird. Hierfür wird erfindungsgemäß ein Messverfahren mit einem Auswerteverfahren gekoppelt, das keine Annahme über den zeitlichen Verlauf der Magenentleerung/Darmabsorption macht und das nachfolgende Prozesse, wie Abbau und Verteilung im Körper, mit einem physiologisch/physikalisch begründeten Modell beschreibt. Parameter, die nicht aus den Messdaten abgeleitet werden können, werden mit empirisch bestimmten Werten belegt, so dass eine robuste Interpretation von Magen-/Darm-Entleerung und Absorbtion und nachfolgender Verstoffwechselung möglich wird.

Mit dem erfindungsgemäßen Test-Verfahren wird der zeitliche Verlauf der oben angegebenen 4 Prozesse, teilweise zusammengefasst und über eine Differentialgleichung beschrieben, die auf einem Kompartiment-(Verteilungsraum)-Modell beruht.

Das erfindungsgemäße Test-Verfahren ermöglicht auch eine erweiterte Anwendung eines $^{13}C$-Atemgas-Tests auf kranke und insbesondere kritisch kranke Patienten, die häufig unter einer Beeinträchtigung der Magen-/Darm-Funktion und/oder der Gallen- und/oder Leberfunktion leiden. Das erfindungsgemäße Testverfahren liefert zuverlässige Ergebnisse, da sowohl der Verlauf der Magen-/Darm-Passage als auch der Anteil der Test-Verbindung, die oxidiert wird, bestimmt werden kann. Eine vollständige Freisetzung des gelabelten Substrats über die $^{13}CO_2$-Abatmung ist nicht erforderlich. Erfindungsgemäß können irreguläre Freisetzungsformen ausgewertet werden, die bislang eine Verwendung von $^{13}C$-Atemgas-Tests verhinderten. Das gesamte Auswertungsverfahren kann vollständig automatisiert werden, so dass ein Anwender nicht eingreifen muss.

Der Begriff "Patient" soll im Rahmen der vorliegenden Erfindung möglichst weitgefasst verstanden werden und bedeutet eine Person, ob krank oder nicht, die dem erfindungsgemäßen $^{13}C$-Atemgas-Test unterzogen wird. Eine Person ohne Erkrankung ist daher eigentlich ein Proband, wird hier aber ebenfalls als "Patient" bezeichnet.

## KURZE BESCHREIBUNG DER ZEICHNUNGEN

Die beigefügten Zeichnungen dienen zur Erläuterung der vorliegenden Erfindung, ohne diese hierauf zu beschränken. Im Einzelnen zeigen:

Figur 1    drei Freisetzungskurven, von denen die oberste eine auswertbare Freisetzungskurve (a) von einer gesunden Person und die weiteren nicht auswertbaren Freisetzungskurven (b) und (c) von kranken Patienten oder Patienten, die Medikamente einnehmen, stammen;

Figur 2    eine Darstellung, die die wesentlichen Elemente des Auswerte-Algorithmus der vorliegenden Erfindung zusammenfasst;

Figur 3    eine schematische Zusammenfassung der Prozesse, die zur Freisetzung von $^{13}CO_2$ in der Atemluft nach Gabe einer Testverbindung führen, als Grundlage der gewählten Modellstruktur zur Herleitung der Gleichungen der vorliegenden Erfindung;

Figur 4a    eine Approximation mit einem Polynom der Ordnung 25 an eine Kurve durch eine Ridge-Regression, basierend auf 15 Messpunkten;

Figur 4b    eine Approximation mit einem Polynom der Ordnung 25 an eine Kurve durch eine Ridge-Regression, basierend auf 40 Messpunkten;

Figur 5    eine Darstellung des Verlaufs der Zielfunktion des MRA (Gleichung 16) und deren Komponenten für verschiedene $\alpha$-Werte (ein Maß für die Biegsamkeit von R(t));

Figur 6    die anhand der Messdaten ermittelten $^{13}CO_2$-Freisetzungskurven gemäß einem Ausführungsbeispiel der vorliegenden Erfindung, wobei die Kurve (1) die Freisetzungskurve in nüchternem Zustand und die Kurve (2) die Freisetzungskurve nach dem Frühstück zeigt; und

Figur 7    die zurückgerechnet optimalen Transfer-Funktionen R(t), basierend auf den gemessenen $\delta$ $^{13}C$-Messdaten von Figur 6, wobei Kurve (1): nüchtern und Kurve (2): nach dem Frühstück darstellt.

## DETAILLIERTE BESCHREIBUNG DER ERFINDUNG

Nachfolgend wird das erfindungsgemäße Verfahren im Einzelnen beschrieben, wobei die Ausführungen für das Verfahren in gleicher Weise für die Vorrichtung gelten sollen und umgekehrt.

### Verabreichung der $^{13}C$-markierten Verbindung

Gemäß dem erfindungsgemäßen Verfahren wird eine mit $^{13}C$-markierte Verbindung an einen Patienten verabreicht, durch den die $^{13}C$-markierte Verbindung zu einem $^{13}C$-markierten Metabolisierungsprodukt umgewandelt wird. Das $^{13}C$-markierte Metabolisierungsprodukt ist $^{13}CO_2$.

Es kommen $^{13}C$-markierte Verbindungen in Betracht, die für den Menschen verträglich sind und diesen nicht nachteilig beeinflussen. Es wird insbesondere eine Verbindung ausgewählt und mit $^{13}C$ markiert, die über den Stoffwechsel zu $^{13}CO_2$ abgebaut wird und die vorzugsweise spezifisch in einem Organ abgebaut wird, wobei das Organ ausgewählt ist aus dem Magen-/Darm-Trakt, der Galle oder der Leber.

Wenn beispielsweise die Entleerung des Magen-/Darm-Trakts überprüft werden soll, wird eine Verbindung markiert und ausgewählt, die vorwiegend im Magen-/Darm-Trakt zu $^{13}CO_2$ metabolisiert wird, so dass das gebildete $^{13}CO_2$ hierauf zurückgeführt werden kann. Dies sind beispielsweise Essigsäure und deren Salze und Derivate, wie Acetate.

Soll die Stoffwechselfunktion eines Patienten überprüft werden, so wird eine Verbindung ausgewählt und mit $^{13}C$ markiert, die hauptsächlich in der Galle oder der Leber verstoffwechselt und zu $^{13}CO_2$ abgebaut wird. Verbindungen, die in der gewünschten Weise hauptsächlich in der Leber verstoffwechselt werden, sind beispielsweise die folgenden Verbindungen: Erythromycin, Methyl-Caffein, als Substrate für Cytochrom P 450 Systeme oder $\alpha$-Keto-Isocapron-Säure für die mitochondriale Oxidation.

Für die Galle sind dies spezielle Triglyceride, bei denen in der Galle die Spaltung der Triglyceride stattfindet oder $^{13}C$-gemischte Triglyceride (etwa aus 2 Stearinsäuren und einer $^{13}C$ markierten Octan-Säure)

Die Testverbindung kann dem Patienten in fester oder flüssiger Form verabreicht werden. Bevorzugt ist eine flüssige Verabreichungsform, damit der Patient diese in einfacher Weise zu sich nehmen kann. Beispielsweise kann die Testverbindung auch einer Nährlösung beigemischt werden, die im Rahmen der klinischen Versorgung verabreicht wird.

Die Testverbindung wird bevorzugt in einer derartigen Menge verabreicht, dass ein sicher messbares $^{13}CO_2$-Signal erzeugt wird, bevorzugt etwa ab 10 $\delta oB$ ($\delta$ over baseline). Hierbei wird der $\delta^{13}C$ -Wert ohne Verabreichung der Testverbindung ($\delta_{basal}$) mit dem $\delta^{13}C$ -Wert nach Verabreichung der Testverbindung ($\delta_{Probe}$) verglichen und man gelangt zu $\delta_{Probe}$ - $\delta_{basal}$ = $\delta oB$. $\delta^{13}C$-Werte entsprechen sehr kleinen $^{13}C$-Anreicherungs-Werten. So sind 1000 $\delta$ (das 1000-fache des $\delta$ $^{13}C$-Werts) ungefähr gleich einer 1%igen Anreicherung gegenüber dem natürlichen $^{13}C$-Vorkommen. Dementsprechend sind natürlich vorkommende Schwankungen der $\delta^{13}C$ -Werte relativ groß und liegen bei etwa 1 $\delta^{13}C$. Daher ist es zweckmäßig, wenn die Signalgröße vorzugsweise mindestens bei etwa 10 $\delta oB$ liegt. Die $\delta$-Werte sind daher die Änderungen der relativen Konzentrationen bzw. Anreicherungsdaten, die als Differenz zu den in der Ausatemluft üblicherweise vorliegenden $^{13}CO_2$-Werten bestimmt werden. Bei Acetat als Testverbindung entspricht die Menge, die ein sicher messbares $^{13}CO_2$-Signal erzeugt, etwa 2 bis 5 mg pro kg Körpergewicht des Patienten. In der Regel werden dem Patienten 2 mg bis 10 mg der Testverbindung pro kg Körpergewicht, bevorzugt auf einmal, verabreicht.

### Ermittlung der Messdaten

Erfindungsgemäß erfolgt unmittelbar nach Verabreichung der Testverbindung die Bestimmung der Änderung der relativen Konzentrationen des $^{13}$C-markierten Metabolisierungsprodukts in der Atemluft des Patienten über ein vorbestimmtes Zeitintervall unter Erhalt von Messwerten. Der Begriff "Änderung der relativen Konzentrationen" oder "Anreicherungsdaten" bedeutet, dass in der Ausatemluft stets $^{13}$C-markiertes $CO_2$ als natürlich vorkommendes Isotop enthalten ist, so dass ein bestimmtes Grundniveau immer vorhanden ist, von dem ausgehend gemessen wird. Direkt nach Verabreichung wird daher die $^{13}CO_2$-Anreicherung im Atemgas des Patienten bestimmt. "Atemgas" bedeutet im Rahmen der Erfindung die ausgeatmete Luft des Patienten. Die Messwerte werden bezogen auf das ausgeatmete Volumen des Atemgases ermittelt, d.h. die Menge die abgeatmet wird. Mit anderen Worten sind die Messwerte in erster Linie $^{13}CO_2$-Anreicherungen, die auf die natürlich vorkommende $^{13}CO_2$-Anreicherung bezogen werden. Eine Umrechnung auf absolut abgeatmete Mengen ist dann möglich (Bezug auf Volumen des Atemgases), wenn die Gesamt-$CO_2$-Produktion mitgemessen wurde.

Das Metabolisierungsprodukt in Form von $^{13}CO_2$, kann durch Einzelmessungen, d.h. diskontinuierlich, oder on-line, d.h. kontinuierlich, durchgeführt werden. Beim diskontinuierlichen Verfahren wird beispielsweise die Patientenprobe als Atemgas in Beuteln zur Messvorrichtung gebracht. Das erfindungsgemäße Testverfahren kann auch kontinuierlich arbeiten, wobei das Atemgas des Patienten dann kontinuierlich mittels einer festen Online-Entnahme aufgenommen werden kann. Die Messung kann in beliebiger Weise unter Verwendung einer Vorrichtung zur Bestimmung des $^{13}CO_2$-Gehalts, vorzugsweise eines $CO_2$-Gasdetektors oder -fühlers durchgeführt werden. Bevorzugt wird unter Verwendung von spektroskopischen Verfahren gemessen, wie IR- oder Laser-spektroskopischen Verfahren, beispielsweise FTIR, Laseranregung bestimmter Rotationslinien oder der NDIR-Messtechnologie (nichtdispersive isotopenselektive Infrarotspektroskopie) (Fischer Analysen Instrumente GmbH, Leipzig), die im Stand der Technik für die Messung von $CO_2$-Gas bekannt sind. Hierfür sind kompakte Messgeräte erhältlich.

### Erfassung der Messdaten

Die Messung erfolgt bevorzugt über eine Dauer von 30 Minuten bis zu 5 Stunden. Es werden mehrere Messwerte in Form von Anreicherungsdaten bestimmt. Beispielsweise können 10 bis 50 Messwerte bestimmt werden; in der Regel ist es jedoch ausreichend, wenn zumindest 10 Messwerte vorliegen, um eine $^{13}CO_2$-Freisetzungskurve zu bekommen, um hieraus eine zurückberechnete optimale Transfer-Funktione R(t) zu erhalten.

Die Messpunkte werden so dicht gelegt, dass eventuelle Änderungen in der Freisetzung von $^{13}CO_2$ erfasst werden können. Die Messdaten werden vorzugsweise solange erhoben, bis etwa 70% oder mehr der $^{13}CO_2$-Menge, die über Oxidation entstanden ist, auch tatsächlich abgeatmet wurde.

### Auswertung der Messdaten

Im Anschluss an die Ermittlung und Erfassung der Messwerte in Form der Änderung der relativen Konzentrationen des $^{13}$C-markierten Metabolisierungsprodukts in der Atemluft des Patienten über ein vorbestimmtes Zeitintervall erfolgt die Auswertung der erhaltenen Messwerte anhand einer Abschätzung des Verlaufs des Transfers der Testsubstanz nach Verabreichung hin zum Ort des Stoffwechsels (Transferfunktion), die eine direkte Aussage über die Magen-/Darm-Entleerung und die Stoffwechselfunktionen ermöglicht.

Die Auswertung beruht darauf, dass die Menge des gebildeten $^{13}CO_2$ proportional zur Menge der verabreichten $^{13}$C-markierten Verbindung ist. Weiterhin wird davon ausgegangen, dass die Menge des gebildeten $^{13}CO_2$ proportional zum Produkt der aktuell am Stoffwechsel-Ort vorliegenden Menge der Testverbindung multipliziert mit einer Geschwindigkeitskonstante (Stoffwechselaktivitäts-Faktor) ist (Kinetik 1. Ordnung). Das Ziel der Auswertung der Messdaten ist die Anpassung von berechneten $^{13}CO_2$-Werten über eine mathematische Abschätzung an die gemessenen $^{13}CO_2$-Werte, um eine Freisetzungskurve mit zuverlässigen Werten zu erhalten.

Die Auswertung der gemessenen Anreicherungsdaten erfolgt hierbei vorzugsweise in automatisierter Form. Erfindungsgemäß wird zum einen aus dem Verlauf der $^{13}CO_2$-Anreicherungsdaten der prozentuale Anteil der Testverbindung, der nach der Magen-/Darm-Entleerung oxidiert wurde, bestimmt; zum anderen wird aus dem Verlauf der $^{13}CO_2$-Freisetzung der Verlauf der Magen-/Darm-Entleerung errechnet. Der Verlauf kann zeitlich hoch aufgelöst werden und dient zur Beurteilung der nachfolgenden weiteren Kriterien:

a) ob eine mehrphasige oder gar völlig irreguläre Entleerung vorliegt;

b) wie schnell die Entleerung des Magens erfolgt und wann in etwa die maximale Entleerung stattfindet; und

c) wie effizient die Testverbindung metabolisiert wird, wenn sie nach Magen-Darm Passage den Ort der Verstoffwechselung erreicht hat.

Der mathematische Ansatz zur parallelen Bestimmung des prozentualen Anteils, der oxidiert wird, und des zeitlichen Verlaufs des Magen-Darm-Transfers soll nachfolgend im Einzelnen beschrieben werden.

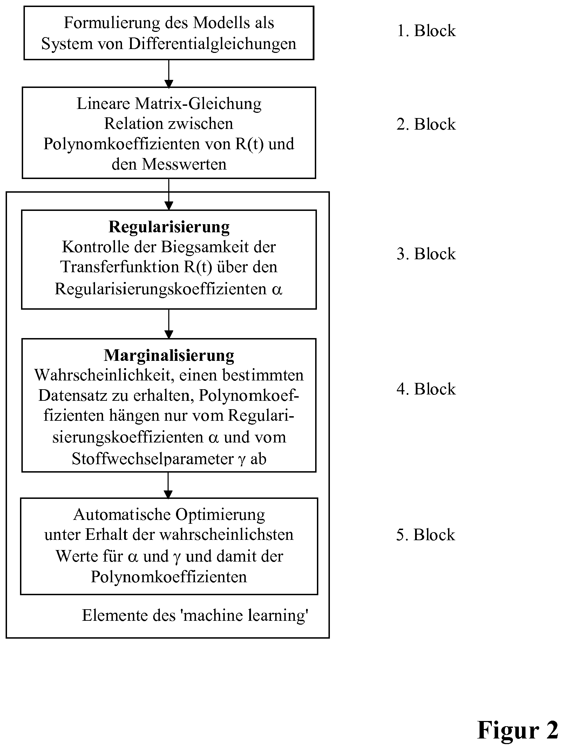

Figur 2 fasst hierzu die wesentlichen Elemente des Auswerte-Algorithmus der vorliegenden Erfindung zusammen:

1. Block: Über die Differentialgleichungen (1) bis (4) wird die Kinetik von $^{13}CO_2$ beschrieben.

2. Block: Die Differentialgleichungen werden in lineare Matrixgleichungen überführt, wobei die erhaltene Gleichung (12) eine Beziehung zwischen Polynomkoeffizienten für die Transfer-Funktion R(t) und den gemessenen Anreicherungsdaten herstellt.

3. Block: Regularisierung: Die Gleichung (13) beschreibt die Kontrolle der Biegsamkeit der Transfer-Funktion R(t) über den Regularisierungskoeffizient $\alpha$.

4. Block: Marginalisierung: Die regularisierte Gleichung (13) wird in eine Wahrscheinlichkeit umgewandelt, dass ein bestimmter Datensatz gemessen wird (siehe Gleichung (15)) und diese Wahrscheinlichkeit wird marginalisiert, so dass nur noch eine Wahrscheinlichkeit berechnet wird, die vom Regularisierungskoeffizienten $\alpha$ und vom Stoffwechselparameter $\gamma$ abhängt, wobei die Polynomkoeffizienten vom Regularisierungskoeffizienten $\alpha$ und dem Stoffwechselparameter $\gamma$ abhängen (siehe Gleichung (14)).

5. Block: automatische Optimierung der Wahrscheinlichkeit unter Variieren des Regularisierungskoeffizienten $\alpha$ und des Stoffwechselparameters $\gamma$ unter Erhalt der wahrscheinlichsten Werte für $\alpha$ und $\gamma$ und damit auch der Polynomkoeffizienten (siehe Gleichung (14)). Hierzu wird ein Minimalwert für die Gleichung (16) mit Hilfe von Standard-Optimierungsverfahren gesucht.

Die einzelnen Blöcke werden nachfolgend im Detail erklärt:

Zeitlicher Verlauf von Magen-Darm-Passage: Transfer-Funktion R(t) (1. Block):

Zunächst werden Gleichungen hergeleitet, mit denen der Verlauf der $^{13}CO_2$-Freisetzung in der Atemluft beschrieben werden kann. Diese Gleichungen können verwendet werden, um die Magen-/Darm-Entleerung und gleichzeitig die oxidative Verwertung der Testverbindung als jeweils getrennt bestimmbare Größen aus der gemessenen $^{13}CO_2$-Anreicherung zu bestimmen.

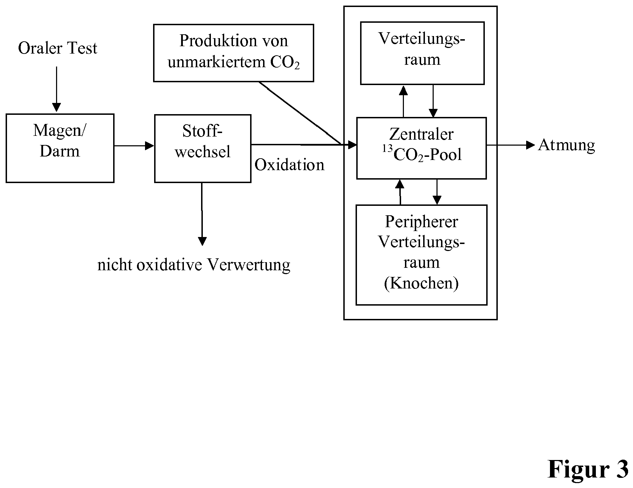

Zur Erläuterung der gewählten Modellstruktur zur Herleitung der Gleichungen wird Figur 3 als Grundlage genommen. In Figur 3 sind in der linken Hälfte die Magenentleerung und der nachfolgende Abbau, am Beispiel von Acetat zu $CO_2$, zu einem Prozess zusammengefasst. Der zeitliche Verlauf wird im Folgenden mit R(t) bezeichnet. R(t) beschreibt den $^{13}CO_2$-Input in das $CO_2$-Verteilungssystem. Das $CO_2$-Verteilungssystem ist im rechten Teil von Figur 3 dargestellt; es beschreibt eine zwischenzeitliche Verteilung von $^{13}CO_2$ im Skelett und verschiedenen Organen und wirkt im Wesentlichen als Verzögerungselement zwischen stoffwechselbedingter, oxidativer $^{13}CO_2$-Produktion und $^{13}CO_2$-Freisetzung über die Atmung.

Figur 3 teilt daher die Prozesse, die zur Freisetzung von $^{13}CO_2$ in der Atemluft nach Gabe einer Testverbindung führen, in zwei Bereiche auf. Der erste Bereich des Modells ist die zeitabhängige Produktion von $^{13}CO_2$ aufgrund von Magenentleerung, Darmpassage und nachfolgender Oxidation. Es wird angenommen, dass die Verwertung des Tracers in Form der $^{13}C$-markierten Testverbindung relativ zu Magenentleerung und Darmtransport sehr schnell abläuft, so dass der zeitliche Verlauf der $^{13}CO_2$-Produktion über die Oxidation im Wesentlichen durch die Magenentleerung und den Darmtransport geprägt wird. Dieser zeitliche Verlauf des Transfers von markiertem $^{13}C$ in die zentrale Zirkulation wird mit R(t) beschrieben. R(t) wird hier als Transfer-Funktion oder Magen-/Darm-Entleerungskurve bezeichnet. Die Verteilungsräume gemäß Figur 3 sind aus Bicarbonat-Kinetikstudien bekannt und wurden hieraus übernommen. Koeffizienten zu deren Beschreibung können nicht aus den Messdaten bestimmt werden. Sie hängen jedoch im Wesentlichen vom Körperbau (Größe, Gewicht, Alter etc.) des Patienten ab und können empirisch bestimmt werden oder sind bereits empirisch bestimmt worden und können als bekannte Größen zur Beschreibung der $^{13}CO_2$-Verteilung in den verschiedenen Räumen verwendet werden.

Für die Transfer-Funktion gelten folgende Überlegungen und Kriterien:

R(t) wird so skaliert, dass das Integral über die Versuchsdauer (Fläche unter der Kurve) gleich 1 ist. Die verabreichte Testdosis wird mit D bezeichnet. R(t)*D ist demnach die Menge, die transferiert wird. Nur ein Teil dieser Menge wird oxidiert, dieser Anteil wird mit einem Splitfaktor f erfasst, der restliche Teil wird für andere Stoffwechsel-Prozesse verwendet. Die Menge an $^{13}CO_2$, die pro Zeiteinheit über die Oxidation produziert wird, ist gleich R(t)*D*f. Das produzierte $^{13}CO_2$ gelangt in die Blut-Zirkulation, von wo aus es sich über den Körper verteilt und über die Lunge abgegeben wird. Die zwischenzeitliche Verteilung von $^{13}CO_2$ in verschiedenen Körper-Kompartimenten und Abgabe über die Lunge wird mit dem Verteilungsmodell beschrieben, das in der rechten Hälfte bzw. dem zweiten Bereich von Figur 3 dargestellt ist. Hiernach ist $y_1$ die $^{13}CO_2$-Menge im Blut, und $y_2$ und $y_3$ sind die $^{13}CO_2$-Mengen in den Verteilungsräumen von Skelett, Darm oder Muskel. Für die zeitlichen Änderungen der $^{13}CO_2$-Mengen aufgrund von Magenentleerung, Stoffwechsel und Verteilungsprozessen gelten daher die nachfolgenden Modellgleichungen (1):

$$\dot{y}_1 = R(t)Df - (k_{01} + k_{21} + k_{31})y_1 + k_{12}y_2 + k_{13}y_3$$
$$\dot{y}_2 = k_{21}y_1 - k_{12}y_2 \qquad (1)$$
$$\dot{y}_3 = k_{31}y_1 - k_{13}y_3$$

Aus dem zentralen Pool, der mit $y_1$ erfasst wird, wird das $^{13}CO_2$ über die Atmung und über die Nieren abgegeben. Messbar ist hierbei die $^{13}CO_2$-Anreicherung in der Atemluft. Diese Anreicherung entspricht der Anreicherung im zentralen Pool $y_1$ und Änderungen der Anreicherung im Blut spiegeln sich direkt in Änderungen der $^{13}CO_2$-Anreicherung in der Atemluft wieder.

System für die $^{13}CO_2$-Anreicherungsdaten

Die obigen Gleichungen im System (1) beziehen sich auf absolute Mengen von $^{13}CO_2$ in den einzelnen Körper-Kompartimenten und werden daher in ein System transformiert, das sich auf die $^{13}CO_2$-Anreicherung bezieht. Hierbei gelten folgende Überlegungen:

Im Körper wird fortlaufend $CO_2$ produziert und über die Atmung abgegeben, wobei der natürlich vorkommende Kohlenstoff immer das stabile $^{13}C$-Isotop in einem Anteil von 0,96 bis 1,2%, bezogen auf 100% Kohlenstoff, enthält. Für die Verteilung des $CO_2$ (egal woher es stammt, ob es aus der Nahrung oder aus der Testverbindung kommt) gilt das Verteilungsmodell, das in der rechten Hälfte von Figur 3 dargestellt ist. In gleicher Weise gilt die obige Gleichung (1), nur dass anstelle von R(t) * D * f die Produktion von $CO_2$, bezeichnet als Gesamt-$CO_2$-Produktion, eingesetzt werden muss. In einer guten Näherung kann angenommen werden, dass dieses System im stationären Gleichgewicht ist, d.h. die Konzentrationen an $CO_2$ ändern sich in den einzelnen Verteilungsräumen nicht. Gemäß Gleichung (1) gilt nun für die Pools von $CO_2$, die mit $x_1$, $x_2$, $x_3$ bezeichnet werden:

$$\dot{x}_1 = 0 \quad ; \quad Gesamt - CO_2 - Produktion = k_{01}x_1$$
$$\dot{x}_2 = 0 \quad ; \quad k_{21}x_1 = k_{12}x_2 \rightarrow x_2 = \frac{k_{21}}{k_{12}}x_1 \qquad (2)$$
$$\dot{x}_3 = 0 \quad ; \quad k_{31}x_1 = k_{13}x_3 \rightarrow x_3 = \frac{k_{31}}{k_{13}}x_1$$

Die Anreicherungsraten sind definiert als: $z_1 = y_1 / x_1$; $z_2 = y_2 / x_2$; $z_3 = y_3 / x_3$; Unter stationären Bedingungen für das unmarkierte System gilt $\dot{z}_1 = \dot{y}_1/x_1$. Demnach werden die einzelnen Zeilen in Gleichung 1 durch $x_1$, $x_2$ bzw. $x_3$ dividiert. Dabei entstehen gemischte Ausdrücke wie $y_1/x_2$. Sie werden durch entsprechende Ausdrücke auf der rechten Seite von Gleichung (2) ersetzt. Aus dem Term, der die Transfer-Funktion enthält wird:

$$\frac{R(t)*D*f}{x_1} = \frac{R(t)*D*f*k_{01}}{Gesamt\text{-}CO_2\text{-}Produktion} = R(t) * \gamma * k_{01} \qquad (3)$$

$$wobei \quad \gamma = \frac{D * f}{Gesamt - CO_2 - Produktion}$$

Mit $z_1$, $z_2$ und $z_3$ als Variablen für die $^{13}CO_2$-Anreicherungen in den einzelnen Kompartimenten erhält man:

$$\dot{z}_1 = (R(t)\gamma - z_1)k_{01} - (z_1 - z_2)k_{21} - (z_1 - z_3)k_{31}$$
$$\dot{z}_2 = (z_1 - z_2)k_{12} \qquad (4)$$
$$\dot{z}_3 = (z_1 - z_3)k_{13}$$

Der Verlauf von $z_1$ gibt den Anreicherungs-Verlauf im zentralen $CO_2$-Pool und in der Atemluft wieder. Dessen Verlauf hängt von der Funktion R(t), von der Verteilung in den verschiedenen Kompartimenten und damit von den Koeffizienten $k_{01}$, $k_{12}$, $k_{21}$, $k_{31}$ und $k_{13}$ ab, die das $CO_2$-Verteilungssystem auf der rechten Seite von Figur 3 charakterisieren. Darüber hinaus hängt es von dem Parameter $\gamma$ ab, der im Wesentlichen die $^{13}CO_2$-Verdünnung durch die

Gesamt-$CO_2$-Produktion im Körper erfasst. Deswegen wird der Stoffwechselparameter $\gamma$ hier auch als Tracer-Verdünnungsfaktor bezeichnet.

Konventioneller Ansatz zur Lösung von Gleichung (4)

Gleichung (4) stellt ein System von miteinander gekoppelten Differentialgleichungen dar. Sie beschreiben die zeitlichen Veränderungen der $^{13}CO_2$-Anreicherungen in den einzelnen Kompartimenten. Zur Lösung eines derartigen Systems wird eine numerische Integration herangezogen. Hierbei wird von einem Ausgangszustand, definiert durch die Konzentrations-Variablen $z_1$, $z_2$ und $z_3$, über Gleichung (4) berechnet, wie sich die Anreicherungen über einen sehr kurzen Zeitraum verändern. Diese Konzentrationsänderung wird den Variablen $z_1$, $z_2$ und $z_3$ zugerechnet und als neuer Status für einen kurzen Zeitpunkt später angenommen. Von diesem neuen Status aus werden erneut nachfolgende Änderungen berechnet. Nach diesem Schema 'tastet' sich der Algorithmus in kleinen Schritten (bis zu 10000) vom Anfangszeitpunkt bis zum Zeitpunkt, der das Versuchsende darstellt. Mit dem vollständigen Durchlauf dieses Algorithmus steht der zeitliche Verlauf der Konzentrationen zur Verfügung. Der berechnete Verlauf kann mit dem tatsächlich gemessenen Verlauf verglichen werden. Unterschiede zwischen Messung und Berechnung werden in einer Fehlernorm erfasst.

Die Größe der Fehlernorm hängt von den Koeffizienten $k_{01}$, $k_{12}$, $k_{21}$, $k_{31}$ und $k_{13}$, dem Parameter $\gamma$, und Hilfsgrößen ab, die den Verlauf von R(t) beschreiben. Um diese für ein einzelnes Experiment zu bestimmen, startet man mit passenden Anfangswerten, berechnet diese für die Fehlernorm, ändert den Wert eines oder mehrerer Koeffizienten und überprüft, ob sich damit die Fehlernorm verbessert, und übernimmt im Erfolgsfall den neuen Koeffizienten-Wert. Auf diese Weise 'tastet' man sich in Richtung eines optimalen Koeffizienten-Satzes vor (als typisches Beispiel siehe: http://de.wikipedia.org/wiki/Quasi-Newton-Verfahren). Dabei gilt, dass für jeden Schritt in der Suche nach optimalen Koeffizienten der Verlauf der Konzentrationen neu berechnet werden muss, was selbst schon viele Einzelschritte verlangt.

Zur Durchführung dieser Schritte gibt es fertige Pakete, z.B. SAAM II (Simulation Analysis and Modeling, The epsilon group, Charlottesville VA, USA), NONMEM (ICON plc -Clinical Research Organisation (CRO) for Drug Development, Dublin Irland), LIXOFT (Modelling & Simulation for Drug Development, Orsay France), so dass das mathematische Programm zur Kopplung der zwei Aufgabengebiete - Bestimmung eines optimalen Satzes von Koeffizienten und numerische Lösung des Differentialgleichungssystem-Parametersatzes - nicht selbst programmiert werden muss.

Es bleiben jedoch folgende Probleme:
Es muss eine Funktion R(t) bestimmt werden, die den Transfer der Testverbindung vom Ort der Verabreichung hin zum Ort der Verstoffwechselung beschreibt. Deren Verlauf ist a priori unbekannt und kann hilfsweise über eine Funktion erfasst werden, die den Transfer an fest gesetzten Stützpunkten vorgibt und dazwischen interpoliert.

Dieser Ansatz bringt jedoch folgende Herausforderungen mit sich: Die Stützpunkte sollten dort dicht gesetzt sein, wo die Transfer-Funktion starke Änderungen aufweist und weniger dicht, wo sie nahezu konstant ist. Eine optimale Positionierung sollte sich daher am aktuellen Messdatensatz orientieren. Für jeden Stützpunkt muss ein Freisetzungswert aus dem Messdatensatz bestimmt werden. Es sollten aber weniger Stützpunkte als Messpunkte verwendet werden, weil sonst die gesamte Information, die in dem Messdatensatz steckt, zur Beschreibung der Freisetzungsfunktion verwendet wird und nichts an Information zur Abschätzung des Verwertungs- oder Verstoffwechselungsprozesses übrigbleibt. Die Anzahl der nötigen Stützpunkte kann über 20 sein, damit steigt aber die Anzahl der unbekannten Größen, die mit der Kurvenanpassung bestimmt werden müssen. Je mehr unbekannte Größen verwendet werden, umso größer ist die Gefahr, dass während der Suche nach optimalen Koeffizienten der Algorithmus bei einem Zwischenwert hängen bleibt, der zwar in nächster Nachbarschaft keinen besseren aufweist, wobei aber weiter entfernt wesentlich bessere Werte liegen.

Dies führt zu einem vielschichtigen Optimierungsproblem und es gibt nur wenige Versuche, Atemgaskurven mit Modellen zu beschreiben, die sich eng am physiologischen Vorbild orientieren. Diese Herausforderungen können in einer Forschungsumgebung vom geschulten Spezialisten gelöst werden, stellen aber ein Hindernis dar, wenn die Auswertung der Messdaten vollständig automatisiert und von ungeschultem Pflegepersonal für einen möglichen Routineeinsatz übernommen werden soll.

MRA (Marginalisierungs- und Regularisierungs-Ansatz):

Die oben genannten Probleme: Positionierung der Stützstellen, Anzahl der Stützstellen und mehr als 20 unbekannte Größen, die über Kurvenanpassung bestimmt werden müssen, können durch den MRA (Marginalisierungs- und Regularisierungs-Ansatz) überwunden werden. Dieser Ansatz wurde bereits in Vogt J. A., Denzer C., Estimation of parameters for the elimination of an orally administered test substance with unknown absorption, Journal of Pharmacokinetics and Pharmacodynamics, 2013; 40(2): 177-187 erläutert. Dort wird gezeigt, dass eine Transfer-Funktion nach oraler Gabe einer Test-verbindung aus dem Konzentrationsverlauf der Testverbindung im Blut rekonstruiert werden kann. Diese Arbeit von Vogt und Denzer bezieht sich jedoch auf die Bestimmungen der Plasma-Konzentration der Testverbindung, die nicht mit der $^{13}CO_2$-Konzentration in der Atemluft gleichgesetzt werden kann. Zudem erfordert dieser Ansatz, dass die Verstoffwechselungs-Rate in der Plasma-Konzentration linear ist, was für biologische Prozesse jedoch häufig nicht zutrifft. Diese Arbeit diente daher nur als technologischer Hintergrund zur vorliegenden Erfindung. Die einzelnen Schritte des Ansatzes werden im nachfolgenden aufgezeigt, danach wird erläutert, wie dieser auf einen $^{13}CO_2$-Atemgas-Test angewandt werden kann.

Nicht-iterative Lösung des Differentialgleichungssystems (2. Block):

Der MRA, auf dem die vorliegende Erfindung basiert, geht von einem 1-Kompartiment-Model aus, wobei z die Konzentration der Testverbindung im Kompartiment ist und deren Blut-Konzentration entspricht, $k_1$ zerfasst das Verschwinden der Testverbindung, etwa durch Abbau, und R(t) erfasst die Freisetzung der Testverbindung nach oraler Gabe. Für die zeitliche Veränderung der Konzentration gilt damit:

$$\dot{z} = -k_1 z + R(t) \tag{5}$$

Betrachtet man die Konzentration zu bestimmten Zeitpunkten oder Stützstellen $t_o,..., t_n$ dann lässt sich Gleichung (5) als Matrixgleichung darstellen (siehe http://en.wikipedia.org/wiki/System_of_linear_equations):

$$\begin{pmatrix} \dot{z}(t_0) \\ \cdots \\ \dot{z}(t_n) \end{pmatrix} = \begin{pmatrix} -k_1 & & \\ & -k_1 & \\ & & -k_1 \end{pmatrix} \times \begin{pmatrix} z(t_0) \\ \cdots \\ z(t_n) \end{pmatrix} + \begin{pmatrix} R(t_0) \\ \cdots \\ R(t_n) \end{pmatrix} \tag{6}$$

Über einen 'collocation-Ansatz' über n-Stützstellen (allgemeine Definition siehe http://en.wikipedia.org/wiki/Collocation_methods) lässt sich der Vektor der zeitlichen Ableitung von z an den Stützstellen als Matrixgleichung darstellen:

$$\begin{pmatrix} \dot{z}(t_0) \\ \cdots \\ \dot{z}(t_n) \end{pmatrix} = (D) \times \begin{pmatrix} z(t_0) \\ \cdots \\ z(t_n) \end{pmatrix} \tag{7}$$

wobei die Matrix D gemäß Mason J.C., Handscomb D.C., Chebyshev polynomials, Chapman & Hall/CRC; 2003, berechnet werden kann. Gleichung (6) lässt sich damit umformulieren zu:

$$\left[ D + \begin{pmatrix} k_1 & & \\ & k_1 & \\ & & k_1 \end{pmatrix} \right] \times \begin{pmatrix} z(t_0) \\ \cdots \\ z(t_n) \end{pmatrix} = \begin{pmatrix} R(t_0) \\ \cdots \\ R(t_n) \end{pmatrix} \tag{8}$$

Damit kann der Zeitverlauf der Konzentration als einfache Matrixgleichung dargestellt werden, die mit Methoden der linearen Algebra gelöst werden kann. Eine zeitaufwändige iterative Lösung entfällt.

Im nächsten Schritt wird die zeitabhängige Funktion R(t) durch ein Polynom ersetzt; wobei $w_o,..., w_m$ die entsprechenden Polynomkoeffizienten sind:

$$R(t) = w_0 + w_1 t + w_2 t^2 + \ldots + w_i t^i + \ldots + w_m t^m \tag{9}$$

m ist der Grad des Polynoms und erfasst die Anzahl der Elemente des Polynoms. Je nach dem Wert der einzelnen Koeffizienten $w_i$ kann ein Polynom eine Auf- oder Abwärtskrümmung nehmen. Die Anzahl der möglichen Krümmungen hängt vom Grad des Polynoms ab und die Intensität der Krümmung wird durch die Größe der Koeffizienten bestimmt. Ersetzen von R(t) mit dem Polynom ergibt für die einzelnen Stützstellen:

$$\begin{pmatrix} R(t_0) \\ \cdots \\ R(t_n) \end{pmatrix} = \begin{pmatrix} \text{polynom} \\ \text{funktion} \\ \text{matrix} \end{pmatrix} \times \begin{pmatrix} w_0 \\ \cdots \\ w_m \end{pmatrix} \qquad (10)$$

Eine Lösung von Gleichung (5) kann damit in der Form dargestellt werden:

$$\left[ D + \begin{pmatrix} k_1 & & \\ & k_1 & \\ & & k_1 \end{pmatrix} \right] \times \begin{pmatrix} z(t_0) \\ \cdots \\ z(t_n) \end{pmatrix} = \begin{pmatrix} \text{polynom} \\ \text{function} \\ \text{matrix} \end{pmatrix} \times \begin{pmatrix} w_0 \\ \cdots \\ w_m \end{pmatrix}$$

In verkürzter, symbolischer Form ist dies:

$$M\vec{z} = \Psi\vec{w} \qquad (11)$$

Gleichung (11) ist die Ausgangsgleichung für den automatisierten Ansatz. Nach z aufgelöst, ergibt sich:

$$\vec{z} = M^{-1}\Psi\vec{w} = M_s\vec{w} \qquad (12)$$

Gleichung (12) stellt einen linearen Zusammenhang zwischen dem Konzentrations-Vektor z und dem Vektor der Polynomkoeffizienten $w_0$,..., $w_m$ dar.

Erfindungsgemäß besteht auch die Möglichkeit anstelle von Polynomen für die Transfer-Funktion R(t) andere Funktionen zu verwenden, die nicht-linear mit der Zeit, aber linear in den Koeffizienten sind (sog. lineare Basisfunktion), so dass aufgrund der Linearität in den Koeffizienten eine Marginalisierung möglich ist.

Regularisierung (3. Block):

Gleichung (12) erlaubt eine Berechnung der Konzentration bei gegebenen Koeffizienten für das Polynom für R(t). In diesem Abschnitt soll angegeben werden, wie aus den gemessenen Konzentrationen die Polynomkoeffizienten und damit R(t) abgeschätzt werden kann.

Es sei $\vec{z_m}$ der Vektor von Konzentrations-Werten, gemessen an denselben Stützstellen wie $\vec{z}$. Die Koeffizienten sollen so gewählt werden, dass der Unterschied zwischen gemessenen und über Polynomkoeffizienten berechneten Konzentrationen minimal wird. Der Unterschied ist: $\vec{z_m} - \vec{z}$, oder mit Gleichung (12): $\vec{z_m} - M_s\vec{w}$. Die Präzision, mit denen die Konzentrationen gemessen werden können, kann für die einzelnen Messpunkte variieren. Deswegen wird der Unterschied zwischen Messung und Berechnung auf den jeweiligen Messfehler bezogen. Es sei $\Sigma$ die Varianz/Kovarianz Matrix der entsprechenden Messfehler. Die Summe der normierten Differenzen ist damit: $(\vec{z_m} - M_s\vec{w})^T \Sigma^{-1} (\vec{z_m} - M_s\vec{w})$. Die Polynomkoeffizienten werden nun so gewählt, dass diese Summe minimal wird, oder, dass sich eine bestmögliche Anpassung von berechneter Konzentration an die Messungen ergibt. Das entsprechende Verfahren wird als Regressionsrechnung oder 'least square' Verfahren (http://de.wikipedia.org/- wiki/Ausgleichungsrechnung) bezeichnet. Wenn die Anzahl der Elemente für das Polynom für R(t) groß genug ist, dann können alle möglichen Krümmungen oder zeitlichen Änderungen in den gemessenen Konzentrationen mit dem Transfer-Polynom R(t) nachgezeichnet werden.

Es besteht jedoch die Gefahr, dass die Transfer-Funktion R(t) so flexibel wird, dass auch zufällige Messfehler in der Konzentration nachgezeichnet werden können. Darüber hinaus kann es schwierig werden, eine große Anzahl von Polynomkoeffizienten aus den Messwerten zu bestimmen. Um diese Probleme in den Griff zu bekommen nützt man aus, dass die Größe der Polynomkoeffizienten proportional zur 'Biegsamkeit' des Polynoms ist: Mögliche Krümmungen von R(t) werden eingeschränkt, indem die (absolute) Größe der Polynomkoeffizienten eingeschränkt wird. Der Fehlerquadratsumme, die den Unterschied zwischen berechneter und gemessener Konzentration erfasst, wird daher eine Größe hinzugefügt, die der durchschnittlichen Koeffizientengröße proportional ist. Dem Betrag nach große Polynomkoeffizienten spielen somit die gleiche Rolle wie Anpassungsfehler. Die Zielfunktion, die im Rahmen einer 'least square' Regression, minimiert wird, ist damit

$$\text{Anpassungs-Fehler}(\vec{w}) = (\vec{z_m} - M_s \times \vec{w})^T \Sigma^{-1} (\vec{z_m} - M_s \times \vec{w}) + \alpha \vec{w}^T \vec{w} \qquad (13)$$

Die Regression wird dabei zu einer 'Ridge'-Regression erweitert (Siehe Hastie T, Tibshirani R, Friedman J. H., The Elements of Statistical Learning, 2. Ausgabe, Springer, 2008). Das Ausmaß der Regularisierung wird daher durch den Regularisierungskoeffizienten $\alpha$, der auch als Ridge-Koeffizient oder Biegsamkeitsfaktor bezeichnet wird, bestimmt. Der Regularisierungskoeffizient $\alpha$ legt fest, wie sehr die Durchschnittsgröße der Polynomkoeffizienten zum Anpassungsfehler beiträgt. Eine Suche nach den Koeffizienten $\vec{w}$, die zu einen möglichst kleinen Anpassungs-Fehler führen, ergibt:

$$\vec{w}_m = A^{-1}\Sigma^{-1}M_s\vec{z}_m; \quad \text{mit} \quad A = M_s^T\Sigma^{-1}M_s + \alpha I \qquad (14)$$

Der Subscript in $\vec{w}_m$ deutet an, dass die Werte aus optimaler Anpassung an Messdaten bestimmt wurden.

Marginalisierung (4. Block):

Gleichung (13) erlaubt die Steuerung der Biegsamkeit von R(t) über eine Veränderung des Regularisierungskoeffizienten $\alpha$. Zu große $\alpha$-Werte führen zu starren Freisetzungsfunktionen und schlechter Anpassung an die Messwerte, zu kleine $\alpha$-Werte zu guter Anpassung und einer Transfer-Funktion R(t) mit 'übersteuerten' Krümmungen.

Im Idealfall sollte die Biegsamkeit gerade so groß sein, dass sie wesentliche Änderungen der Magenentleerung/Darmpassage erfassen kann, ohne dass Messfehler in der Konzentration kleinere Krümmungen oder Dellen im Verlauf von R(t) produzieren.

Um einen guten Kompromiss zwischen beiden Extremen zu erzielen, müsste man $\alpha$-Werte suchen, die die Anpassungsnorm in Gleichung (13) minimieren. Dabei würde jedoch $\alpha$ gegen Null gehen, mit übermäßigen Fluktuationen in R(t). Gleichung (13) kann jedoch verwendet werden, um ein Produkt von Wahrscheinlichkeiten zu formulieren, dass ein bestimmter Datensatz, in Abhängigkeit vom Regularisierungskoeffizienten $\alpha$, vom Stoffwechselparameter $\gamma$, von (z.B. aus der Literatur bekannten) kinetischen Koeffizienten und von Polynomkoeffizienten beobachtet bzw. gemessen wird. Diese Wahrscheinlichkeit ist:

$$Q \sim P(\vec{z_m} \mid k_{01}..k_{31}, \vec{w}, \alpha, \gamma) * P(\vec{w}, \alpha) \qquad (15)$$

Das Besondere dabei ist, dass Q in $\vec{w}$ und $\vec{z_m}$ normal verteilt ist. Damit können, durch formale Integration über alle möglichen Polynomkoeffizienten, diese heraus integriert werden, so dass eine Wahrscheinlichkeit verbleibt, einen Messdatensatz zu finden, bei bekannten kinetischen Koeffizienten, der nur vom Regularisierungskoeffizienten' $\alpha$ und dem Stoffwechselparameter $\gamma$ abhängt. Gemäß einer erfindungsgemäßen Variante kann Q auch um eine Wahrscheinlichkeit für die kinetischen Koeffizienten erweitert werden.

Zur Parameterbestimmung wird der negative Logarithmus dieser Wahrscheinlichkeit herangezogen, dieser ist:

$$-ln(Q) \sim \text{Anpassungs-Fit norm}(\vec{w}_m) + ln \mid A \mid -m\,ln(\alpha) \qquad (16)$$

wobei $\vec{w}_m$ Polynomkoeffizienten sind, die über Gleichung (14) berechnet wurden, und $m$ ist der Grad des Polynoms für R(t). Über die Anpassungs-Fitnorm hängt die Gleichung (16) auch von $k_{01} ... k_{31}$, $\gamma$ ab. Gleichung (16) ist die zentrale Gleichung des MRA. Aus den gemessenen $^{13}CO_2$-Atemgas-Freisetzungskurven kann somit der Faktor $\gamma$, der Regularisierungskoeffizient $\alpha$ und die Magen-/Darm-Entleerungskurve oder Transfer-Funktion R(t) bestimmt werden.

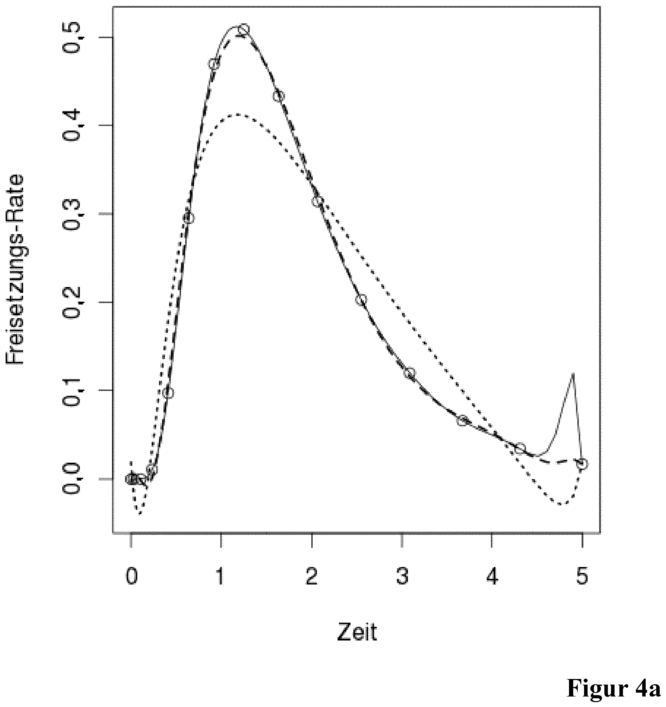

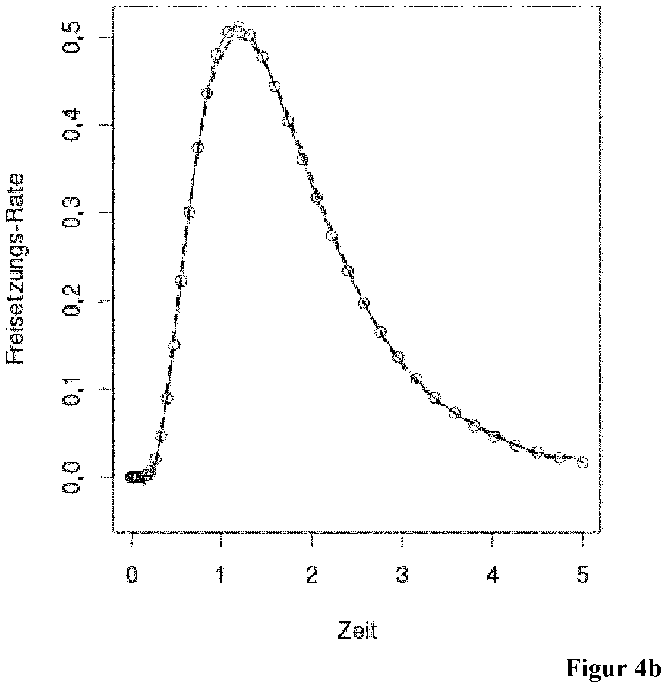

Figur 4a und Figur 4b zeigen für den Fall der Anpassung eines Polynoms an eine Kurve, wie über eine Ridge-Regression eine gute Anpassung erzielt werden kann. Um verschiedene mögliche Kurvenverläufe zu erfassen ist der Grad des Polynoms (Anzahl der Komponenten) möglichst hoch. Die Darstellungen zeigen jeweils eine Kurve, die über ein Polynom approximiert werden soll, anhand von 15 Messpunkten (Figur 4a) oder 40 Messpunkten (Figur 4b), mit dichter gelegten Messpunkten am Anfang der Kurve. In Figur 4a stellt die Kurve mit durchgezogener Linie eine Polynom-Approximation mit minimalem Regularisierungsfaktor $\alpha$ dar, die lang-gestrichelte Line ist eine Polynom-Approximation mit optimalem Regularisierungsfaktor $\alpha$ und die gepunktete Linie ist eine Polynom-Approximation bei zu großem Regularisierungsfaktor $\alpha$. In Figur 4b ist die durchgezogene Linie eine Polynom-Approximation mit minimalem Regularisierungsfaktor $\alpha$ und die lang-gestrichelte Linie eine Polynom-Approximation mit optimalem Regularisierungsfaktor $\alpha$. Die offenen Kreise zeigen Punkte der Kurve, die approximiert werden soll. Diese zeigen den Unterschied zwischen erwarteten und tatsächlich gemessenen Funktionswerten, stellen also eine Messfehler-bedingte Abweichung dar. Die Koeffizienten der Polynome wurden mit einer Gleichung, die Gleichung (14) entspricht, berechnet und daraus mit Gleichung (9) die Polynom-Werte für verschiedene x-Werte berechnet. Die durchgezogene Linie in Figur 4a und 4b bezieht sich auf ein Polynom mit minimaler Regularisierung. Bei 15 Messpunkten ergibt sich ein Überschwingen des Polynomes gegen Ende des Verlaufs. Dieses Überschwingen kann mit einer erhöhten Regularisierung unterdrückt werden, wie die gestrichelte Kurve zeigt. Bei weiter erhöhter Regularisierung ergibt sich eine zu 'steife' Kurve, die den tatsächlichen Verlauf nicht erfassen kann (siehe gepunktete Kurve in Figur 4a) bei 15 Messpunkten. Figur 4a und 4b machen deutlich, dass zur ausreichenden Approximation einer Kurve der Grad eines Polynom bis zu 25 sein kann, und dass es einen Grad der Regularisierung (Größe von $\alpha$) gibt, bei dem die Annäherung des vorgegebenen Kurvenverlaufs optimal ist. Weiterhin ist aus Figur 4b zu entnehmen, dass bei genügend dicht gesetzten Messpunkten die Regularisierung nicht mehr benötigt wird.

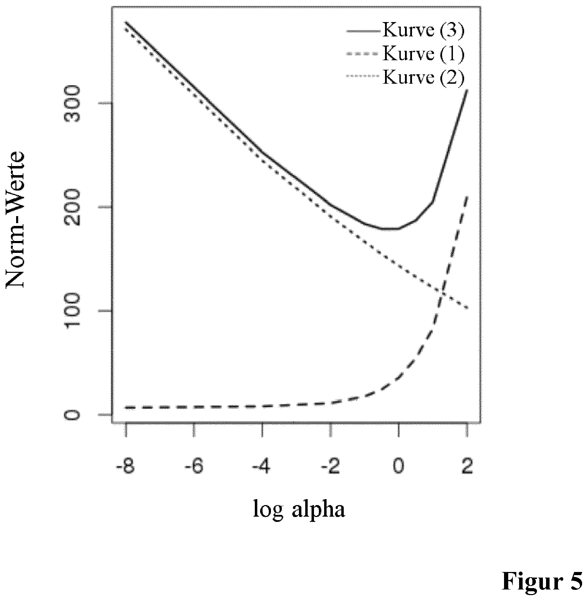

Figur 5 zeigt eine Darstellung des Verlaufs der Zielfunktion (Gleichung 16) des MRA und deren Komponenten für verschiedene $\alpha$-Werte (ein Maß für die Biegsamkeit von R(t)). Die Kurve (1) repräsentiert den 1. Teil von Gleichung (16), die Kurve (2) repräsentiert den 2. Teil von Gleichung (16) und die Kurve (3) stellt die Summe der Kurven (1) und (2) dar. Figur 5 zeigt daher, dass die Gleichung (16) bei einem bestimmten $\alpha$-Wert ein Minimum hat (Kurve (3)). Der optimale Grad der Regularisierung kann daher automatisch über eine Minimierung der Zielfunktion des MRA bestimmt werden. Mit anderen Worten hat diese Funktion einen Minimalwert, der automatisiert bestimmt werden kann. Daraus folgt, dass das optimale Maß der Biegsamkeit von R(t) automatisch bestimmt werden kann.

Für das einfache Modell, das in Gleichung (5) definiert wurde, werden 25 Polynomkoeffizienten, 1 kinetischer Koeffizient und ein $\alpha$-Wert bestimmt. Der rechnerische Aufwand zur Bestimmung von Parametern ist ungefähr proportional zum Quadrat der Anzahl der Parameter. Vergleicht man den Aufwand, der mit dem weiter oben beschriebenen konventionellen Ansatz verbunden ist, mit dem Aufwand für den MRA, dann ergibt sich ein Aufwands-Verhältnis von $27*27$ für den konventionellen Ansatz gegenüber $2*2$ für den MRA oder etwa 180:1.

Wenn die Anzahl der Messpunkte größer ist als der Grad des Polynoms, kann - wie bereits erläutert - die Regularisierung wegfallen. Es ist jedoch so, dass im Einzelfall entschieden werden muss, ob die Regularisierung entfallen kann. Wenn die Anzahl der Messwerte größer ist als der Grad des Polynoms kommt es darauf an, wie viel größer die Anzahl der Messwerte ist. Um dies anschaulich zu erklären: Es kann der Fall eintreten, dass die Anzahl der Messwerte zwar größer ist als der Grad des Polynoms, jedoch einzelne Messwerte so weit voneinander entfernt liegen, dass die errechnete Kurve zwischen den Werten nach oben und unten "durchschwingt", d.h. die Messwerte werden nicht direkt miteinander verbunden (siehe hierzu auch Figur 4a). In diesem Fall ist es zweckmäßig, wenn eine Regularisierung durchgeführt wird.

Anpassung des Atemgas-Modells an den MRA (5. Block):

Der in den Gleichungen (5) bis (16) beschriebene MRA wurde für ein einfaches System hergeleitet, bei dem eine unbekannte Freisetzungsfunktion in ein einzelnes Kompartiment mündet, wo die Konzentration der Testverbindung messbar ist. Mit den Gleichungen (1) bis (4) wurde ein System aufgestellt, um die Situation eines $^{13}CO_2$-Atemgas-Tests zu erfassen (siehe 1. Block). Um auf dieses $^{13}CO_2$-System den MRA anwenden zu können, wird es in der Matrix-Form dargestellt (siehe 2. Block). Es sind $\vec{z_1}, \vec{z_2},$ und $\vec{z_3}$ Vektoren, die $^{13}CO_2$-Anreicherungen in den einzelnen Kompartimenten zu den verschiedenen Zeitpunkten darstellen. Es gilt:

$$M_{11} = \left[ D + \begin{pmatrix} k_{01} + k_{21} + k_{31} & & \\ & k_{01} + k_{21} + k_{31} & \\ & & k_{01} + k_{21} + k_{31} \end{pmatrix} \right]$$

$$M_{22} = \left[ D + \begin{pmatrix} k_{12} & & \\ & k_{12} & \\ & & k_{12} \end{pmatrix} \right]; M_{33} = \left[ D + \begin{pmatrix} k_{13} & & \\ & k_{13} & \\ & & k_{13} \end{pmatrix} \right]; \tag{17}$$

Die Teilmatrix D ist in Gleichung (7) definiert. Für das 3-Kompartiment-System zur Beschreibung der $^{13}CO_2$-Kinetik ergibt sich damit folgende entsprechende Block-Matrix-Gleichung: (siehe http://de.wikipedia.org/wiki/Blockmatrix)

$$\begin{pmatrix} M_{11} & M_{21} & M_{31} \\ M_{12} & M_{22} & \\ M_{13} & & M_{33} \end{pmatrix} \times \begin{pmatrix} \vec{z_1} \\ \vec{z_2} \\ \vec{z_3} \end{pmatrix} = \begin{pmatrix} k_{01}\gamma\Psi\vec{w} \\ 0 \\ 0 \end{pmatrix} \tag{18}$$

wobei $M_{12}, M_{13}, M_{21}$ und $M_{31}$ Diagonal-Matrizen mit den jeweiligen Elementen $k_{12}, k_{13}, k_{21}$ und $k_{31}$ sind. Aus Gleichung

(18) kann $\vec{z}_2$ und $\vec{z}_3$ über Block-Matrix-Operationen eliminiert werden, und es ergibt sich ein Ausdruck, der sich nur auf $\vec{z}_1$ bezieht:

$$\left[ M_{11} - (M_{12} \quad M_{13}) \times \begin{pmatrix} M_{22} & \\ & M_{33} \end{pmatrix}^{-1} \times \begin{pmatrix} M_{21} \\ M_{31} \end{pmatrix} \right] \vec{z}_1 = k_{01} \gamma \Psi w \tag{19}$$

Gleichung (19) hat dieselbe Struktur wie Gleichung (11) nur dass die Struktur der Matrix auf der linken Seiten komplexer ist. Da Gleichung (11) als Ausgangspunkt für die MRA-Optimierungsverfahren gilt, kann Gleichung (19) ebenso für diese Zwecke verwendet werden.

Bestimmung unbekannter Größen des $^{13}CO_2$-Systems

Nach Umwandeln der regularisierten Gleichung (Gleichung (13)) in eine Wahrscheinlichkeit, dass ein bestimmter Datensatz in Abhängigkeit vom Regularisierungskoeffizienten a, den kinetischen Koeffizienten, einem Stoffwechselparameter $\gamma$ und den Polynomkoeffizienten gemessen wird (Gleichung (15)) wird die Marginalisierung der Wahrscheinlichkeit durchgeführt, so dass nur eine Wahrscheinlichkeit berechnet wird, die vom Regularisierungskoeffizienten a, und vom Stoffwechselparameter $\gamma$ abhängt, wobei die Polynomkoeffizienten vom Regularisierungskoeffizienten $\alpha$ und dem Stoffwechselparameter $\gamma$ abhängen (Gleichung (14)).

Dann kann die automatische Optimierung der Wahrscheinlichkeit unter Variieren des Regularisierungskoeffizienten $\alpha$ und des Stoffwechselparameters $\gamma$ unter Erhalt der wahrscheinlichsten Werte für $\alpha$ und $\gamma$ und damit auch der Polynomkoeffizienten erfolgen. Hierzu werden für einen Satz an $^{13}CO_2$-Anreicherungsdaten Werte für den Regularisierungsfaktor $\alpha$ und den Stoffwechselparameter $\gamma$ über iterative Verfahren gesucht, die einen Minimalwert für Gleichung (16) ergeben. Für jeden Schritt im iterativen Verfahren wird für gegebenes $\alpha$ und $\gamma$ über die Gleichungen (17) bis (19) die Matrix $M_s$ berechnet, die dann in Gleichung (14) verwendet wird, um die Polynomkoeffizienten abzuschätzen, die zur Berechnung der (logarithmierten) Wahrscheinlichkeit in Gleichung (16) herangezogen werden. Mit dieser Einbettung der Zwischenschritte werden auch die Polynomkoeffizienten abgeschätzt, über die dann mit Gleichung (9) der Verlauf von R(t) bestimmt werden kann.

Mit der Umwandlung eines $^{13}CO_2$-Systems, das sich auf die Menge in den verschiedenen Kompartimenten bezieht, auf ein System, das sich auf die $^{13}CO_2$-Anreicherung bezieht, wurde:

$$\gamma = \frac{Df}{Gesamt\text{-}CO_2\text{-}Produktion}$$

eingeführt. Der Stoffwechselparameter $\gamma$, der auch als Tracer-Verdünnung bezeichnet wird, beschreibt, wie die Anreicherung des $^{13}C$-markierten Metabolisierungsprodukts $^{13}CO_2$ durch die körpereigene $CO_2$-Produktion verdünnt wird und zum messbaren $^{13}CO_2/^{12}CO_2$-Verhältnis im Atemgas führt. Die Stoffechselparameter $\gamma$ entspricht daher dem eingebrachten Verhältnis von markiertem $CO_2$ zu unmarkiertem $CO_2$ (oder $^{13}CO_2/^{12}CO_2$-Verhältnis) im $CO_2$-Verteilungs-System, das als rechter Bereich in Figur 3 dargestellt ist. Dieses kann aus den Messdaten über eine Optimierung von Gleichung (16) zusammen mit dem Regularisierungsfaktor $\alpha$ bestimmt werden. Aus Gleichung (3) ergibt sich daher:

$$f = \gamma \frac{Gesamt - CO_2 - Produktion}{D} \tag{20}$$

Aus dem abgeschätzten Stoffwechselparameter $\gamma$ kann damit der Splitfaktor f berechnet werden, wenn die Gesamt-$CO_2$-Produktion pro Zeiteinheit des Patienten bekannt ist. Die Gesamt-$CO_2$-Produktion kann mit einer Zusatzeinrichtung direkt beim Patienten erfasst werden, die im Wesentlichen das Atemvolumen pro Zeiteinheit und die Durchschnittskonzentration von $CO_2$ in der Atemluft misst. Die Gesamt-$CO_2$-Produktion kann auch aus Patientenparametern, wie Alter, Gewicht etc. abgeschätzt werden.

Der Splitfaktor erfasst als entscheidende Stoffwechselgröße wie viel von der Testverbindung, die über die Magen-/Darm-Passage in stoffwechselaktive Bereiche gelangt, auch tatsächlich oxidiert wird. Damit kann generell die Stoffwechselaktivität von der Magen-/Darm-Passage getrennt werden. Durch den Splitfaktor gelingt es, ein Maß für die organspezifische Abbaukapazität, insbesondere Leber- und/oder Gallenfunktion eines Patienten zu bekommen. Der Splitfaktor liegt zwischen 0 und 1. Wird die gesamte Menge der Testverbindung, die stoffwechselaktive Bereiche erreicht, oxidiert, ist der Splitfaktor = 1. Splitfaktor = 0 bedeutet, die Testverbindung wird nicht oxidiert. Stoffwechselaktive Bereiche sind Zellen, Gewebe oder Organe, die über die Blutzirkulation zugänglich sind, und in denen die entscheidenden Schritte zur Umwandlung der Testsubstanz in $^{13}CO_2$ ablaufen. Dies ist bei vielen Testverfahren die Leber. Bei einem Test auf die Gallenfunktion müssen im Darm zuerst Fette (Triglyceride) in freie Fettsäuren gespalten werden, damit sie über die Darmzellen aufgenommen werden können und in der Leber weiterverarbeitet werden können. In diesem Fall ist der Darm das entscheidende Organ. Kleine Splitfaktoren im Bereich zwischen 0 und 0,2 bedeuten daher in diesem Zusammenhang eine mangelhafte Spaltung der Triglyceride, keine Aufnahme und Verlust oder Abgabe über den Fäzes, d.h. eine schlechte Verstoffwechselung und damit schlechte Gallen- und/oder Leberfunktion. Dies führt zu niedrigen Splitfaktoren. Beispielsweise wenn ein Patient Fieber hat, können kleine Splitfaktoren erhalten werden. Große Splitfaktoren, beispielsweise größer als 0,6, bedeuten eine erhöhte Verstoffwechselung, die über dem normalen Niveau liegt und daher ebenfalls auf eine Erkrankung hindeuten kann.

Die Interpretation des Splitfaktors für Acetat als Testverbindung ist schwierig: Bei hoher Stoffwechselrate kann die Anreicherung des markierten Testsubstrates verdünnt werden, und gleichzeitig kann das Testsubstrat vermehrt für andere Zwecke, wie etwa die Synthese verwendet werden. Im Falle von Acetat als Testverbindung ist der Splitfaktor f bzw. $\gamma$ nur ein Korrekturfaktor, der notwendig ist um die Gesamthöhe der $^{13}CO_2$-Anreicherung im Atemgas korrekt zu interpretieren. Im Falle des Triglycerid-Testes hat der Splitfaktor an sich eine biologisch/medizinisch wichtige Bedeutung. In diesem Zusammenhang wird z.B. auf die Verstoffwechselung von Acetat verwiesen, bei der der vorliegende Splitfaktor im engen Zusammenhang mit der "Acetate-Recovery" steht, die den prozentualen Anteil an markierten Acetat-Kohlenstoffen angibt, der bei einem Atemgastest im Atemgas Hierzu gibt es zahlreiche Literatur im Stand der Technik.

Durch Auswahl einer entsprechenden Testverbindung, wie bereits beschrieben, die beispielsweise hauptsächlich über die Leber oder die Galle zu $^{13}CO_2$ abgebaut wird, können anhand des Splitfaktors direkte Aussagen über deren Funktion getroffen werden.

Die kinetischen Koeffizienten $k_{01},...k_{13}$ für das $CO_2$-Verteilungssystem sind ein Maß für die Verweilzeit der Testverbindung im Körper, da diese nach der Verstoffwechselung nicht unmittelbar wieder durch den Atem ausgeschieden wird. Sie sind aus dem Verlauf der $^{13}CO_2$-Anreicherung in der Atemluft nicht bestimmbar. Für sie müssen Abschätzungen aus früheren Studien verwendet werden, wie sie etwa in Saccomani M.P., Bonadonna R.C., Caveggion E., DeFronzo R.A., Cobelli C., Bicarbonate kinetics in humans: identification and validation of a three-compartment model, The American Journal of Physiology, 1995, 269(1 Pt I): E183-92 angegeben sind.

Die Bestimmung der kinetischen Koeffizienten kann erfindungsgemäß auch zusätzlich optimiert werden. Hierzu können nicht nur die Regularisierungskoeffizienten $\alpha$ und der Stoffwechselparameter $\gamma$ - wie bereits erläutert - variiert und optimiert werden, sondern zusätzlich können auch die kinetischen Koeffizienten variiert und optimiert werden, jedoch mit der Einschränkung, dass die resultierenden Werte sich im Bereich von bekannten Wahrscheinlichkeitsverteilung für einen kinetischen Koeffizient befinden. Die kinetischen Koeffizienten werden in der Literatur und durchgeführten Studien anhand von Wahrscheinlichkeitsverteilungen bestimmt (Prior im Sinne einer Bayes-Statistik sind vorher bekannte Wahrscheinlichkeitsverteilungen für unbekannte Koeffizienten), so dass deren Wahrscheinlichkeitsverteilungen bekannt sind. Daher werden die kinetischen Koeffizienten oder Stoffwechselkoeffizienten, die sich auf das System zur Verteilung von $CO_2$ im Organismus beziehen, vorzugsweise basierend auf dem bereits durch Studien bekannten Wertebereich, anhand eines sog. Bayesian -Approach entsprechend bestimmt.

Ein zentrales Element der Erfindung ist, dass die Freisetzungskurve über eine biegsame Kurve dargestellt wird, so dass ein optimaler Grad der Biegsamkeit nach einem Ansatz des 'machine learning', eine Variante der künstlichen Intelligenz, berechnet werden kann (s. hierzu auch Bishop C. M., Pattern Recognition and Machine Learning (Information Science and Statistics), 1. Ausgabe, Springer; 2007). Im Bereich des "machine learning" wird für das Problem der optimalen Kurvenbestimmung aus Messdaten ein statistisch motivierter "bester" Kompromiss zwischen Güte der Anpassung und Zuverlässigkeit der Interpolation beschrieben und Formeln für dessen eindeutige Bestimmung hergeleitet, so dass das Optimum ohne Interaktion mit dem Anwender gefunden wird. Dieses automatische Optimierungs-Konzept soll auch erfindungsgemäß implementiert werden. Optimierung per "machine learning" stellt also einen Anwendungsbereich dar, in dem ähnliche Lösungen wie bei der Erfindung verwendet werden.

Der entsprechende Ansatz entwickelt daher eine Formel für die Wahrscheinlichkeit, dass ein bestimmter Messdatensatz bei gegebener Modellstruktur auftritt. Diese Wahrscheinlichkeit hängt nur von einem Parameter für die Biegsamkeit, die dem Regularisierungskoeffizienten $\alpha$ entspricht, und von einer Reaktionskonstante ab, die dem $\gamma$-Wert in diesem Ansatz entspricht. Diese Größen können effizient berechnet werden. Die Koeffizienten für R(t) werden dabei, wie bereits beschrieben, als Zwischenschritt berechnet. Ein entscheidendes Element dieses Auswertungs-Ansatzes ist, dass eine optimale Biegsamkeit berechnet werden kann, ohne dass ein 'subjektiver' steuernder Eingriff eines Anwenders notwendig ist.

Es wird daher eine parallele Ermittlung jeweils von R(t) und $\gamma$ (Verdünnung des $^{13}CO_2$, das durch Oxidation entsteht, durch unmarkiertes $CO_2$ aus allen anderen Oxidationsprozessen, siehe Gleichung (3)) durchgeführt, woraus dann f (der Anteil der Testverbindung, der oxidiert wird) bestimmt werden kann. Ziel der Erfindung ist es, dass diese Größen zusammen mit einer Transfer-Funktion R(t), die den Zeitverlauf der Magen-/Darm-Passage beschreibt, aus den

gemessenen $^{13}CO_2$-Anreicherungsdaten bestimmt werden können.

Das geschilderte Vorgehen ist erfindungsgemäß unabhängig davon, ob die Magen-/Darm-Entleerung normal, das heißt, mit einem Maximalwert, oder mehrphasig ist, wie es bei kranken Patienten sein kann. Die gemessenen $^{13}CO_2$-Anreicherungsdaten dienen daher als Grundlage zur Berechnung der Magen-/Darm-Passage, Kurvenbiegsamkeit (Regularisierungskoeffizient a) und Stoffwechselparameter $\gamma$ anhand der erfindungsgemäß entwickelten mathematischen Abschätzung. Die Berechnungen erfolgen dabei wie bereits im Einzelnen erläutert.

Die errechneten Transfer-Funktionen R(t) haben in der Regel einen engen Konfidenz-Bereich, beispielsweise einen 95%-Konfidenz-Bereich. Dasselbe gilt für den Stoffwechselparameter $\gamma$, d.h. diese Größen können mit einer Präzision von etwa 5% des Nominal-Wertes erfasst werden. Die 5% resultieren in der Unsicherheit, die aus dem Messfehler und der limitierten Anpassung der berechneten an die gemessenen $^{13}CO_2$-Werte entsteht.

Damit ist die Bestimmung von R(t) unabhängig vom Stoffwechsel möglich. Der Anteil, der oxidiert wird, kann aus dem Stoffwechselparameter $\gamma$ berechnet werden, wenn eine Abschätzung der Gesamt-$CO_2$-Produktion vorliegt (siehe Gleichung (20)). Aus $\gamma$ kann der Splitfaktor f berechnet werden, der als Maß für die Stoffwechselfunktionen herangezogen werden kann.

Das erfindungsgemäße Verfahren kann auch bei künstlich beatmeten Patienten eingesetzt werden, dies setzt aber in der Regel eine einfache Anpassung der Messvorrichtung voraus. So ist es hierfür zweckmäßig, wenn das Atemgas von dem Schlauchteil, über das vom Beatmungsgerät die abgeatmete Luft in die Umgebung abgegeben wird, abgegriffen und über eine direkte Verbindung dem Messgerät zugeführt wird. Dies kann beispielsweise durch einen Y-Schenkel oder dergleichen erfolgen. Eventuell kann hierbei eine $O_2$-Korrektur zur Kompensation des Sauerstoffanteils bei der künstlichen Beatmungsluft mit erhöhter Sauerstoffkonzentration vorgenommen werden (s. J.A. Vogt, W. Fabinski, J. Kappler, H. Fischer, M. Georgieff, "Response surface calibration of 13CO2-NDIR offset values: A 'random coefficients' approach" Chemometrics and Intelligent Laboratory Systems, Bd. 107, Nr. 2, Juli 2011, S. 377-383). Dies ist ein entsprechendes Kalibrierungsverfahren, um verfälschende Einflüsse des Trägergases in Form von $O_2$ auszuschließen.

Die Kopplung von Messtechnik und automatisierter Auswertung führt zu zuverlässigen, reproduzierbaren Ergebnissen, die den behandelnden Arzt bei seiner Diagnose unterstützen können.

Überraschenderweise ist eine vollständige Freisetzung von $^{13}CO_2$ jedoch mit dem erfindungsgemäßen Verfahren nicht nötig, wie durch eine Simulation und anhand von durchgeführten Versuchen belegt werden konnte. Es ist erfindungsgemäß ausreichend, wenn die Magen-/Darm-Passage vollständig abgeschlossen ist. Das über die Oxidation freigesetzte $^{13}CO_2$ muss noch nicht vollständig abgeatmet sein, dessen Retention in den einzelnen Verteilungsräumen ist in der Auswertung bereits berücksichtigt.

In einer durchgeführten Simulation wurde Gleichung (1) mit einem konventionellen Programm (http://commons.apache.org/proper/commons-math/userguide/ode.html) zur numerischen Lösung von Gleichung (1) für einen vorgegebenen mehrphasigen Verlauf der Transfer-Funktion und einen vorgegebenen Splitfaktor gelöst. Dabei wurde eine theoretische $^{13}CO_2$-Freisetzung berechnet, aus der synthetische Messdaten erzeugt wurden. Aus diesen synthetischen $^{13}CO_2$-Freisetzungsdaten wurden dann erfindungsgemäß die Transfer-Funktion und der Splitfaktor rekonstruiert, d.h. zurückgerechnet. Aus dem Vergleich zwischen vorgegebenen und rekonstruierten Werten kann man die Güte des Verfahrens abschätzen. Die Simulation war erfolgreich, die Qualität des erfindungsgemäßen Verfahrens ist ausgezeichnet.

## Vorrichtungen zur Durchführung des Verfahrens

Die Erfindung betrifft ebenfalls eine Vorrichtung zur Durchführung des Verfahrens, umfassend

- eine Messvorrichtung zur Bestimmung der Änderung der relativen Konzentrationen des $^{13}C$-markierten Metabolisierungsprodukts in Form von $^{13}CO_2$ in der Atemluft des Patienten über ein vorbestimmtes Zeitintervall unter Erhalt von Anreicherungsdaten;
- Vorrichtungen zur Entnahme der Gasprobe, insbesondere eine Pumpe, Filter, Ventile und Gaswege in Form von Schläuchen aus Gummi, Kunststoff und/oder Metall, mit oder ohne Beheizung;
- optional eine Vorrichtung zur Durchführung von Plausibilitätstests durch Aufgabe von Referenzgasen jederzeit über Ventile in den Messgasstrom;
- Vorrichtungen für eine interne Spannungsversorgung;
- Kommunikationseinrichtungen zur Übertragung von Mess- und Steuerdaten und
- eine Auswertevorrichtung, die derart eingerichtet ist, dass sie die erhaltenen Anreicherungsdaten anhand der mathematischen Abschätzung, die zuvor dargelegt wurde, auswertet.

Geeignete Messvorrichtungen, mit denen die entsprechenden Messungen durchgeführt werden können, gehen beispielsweise aus Modak A., Stable isotope breath tests in clinical medicine: a review, J. Breath Res. 2007; 1:014003 hervor. Beispielsweise genannt werden Messgeräte von Fischer Analysen Instrumente GmbH, Leipzig oder Messgeräte, wie diese in der WO 2007/107366 A1 beschrieben sind.

Die Messvorrichtung umfasst vorzugsweise eine Einrichtung zur Kalibrierung von $O_2$- und $CO_2$, um zusätzliche Effekte auf die $^{13}CO_2$-Anreicherungsmessung auszuschließen, und eine Einrichtung zur Steuerung des Hin- und Abtransport des Atemgases und von Kalibierungsgas zur Messvorrichtung.

Die optionale Vorrichtung zur Durchführung von Plausibilitätstests durch Aufgabe von Prüfgasen jederzeit über Ventile in den Messgasstrom ist eine Vorrichtung, mit der nach dem Aufbauen, Kalibrieren und Justieren der Vorrichtung zur Durchführung des vorliegenden Verfahrens ein Gas in die Vorrichtung eingebracht wird, das eine bekannte Konzentration von $^{13}CO_2$ aufweist, um zu überprüfen, ob die Vorrichtung richtig arbeitet. Dieses Gas, das für einen Plausibiltätstest eingesetzt wird, wird hier auch als Kalibriergas oder Referenzgas bezeichnet.

Durch die erfindungsgemäße Vorrichtung kann beispielsweise im Zeitraum von 2 Min. ein Messwert erhalten werden. Beispielsweise werden zumindest 10 Messwerte bestimmt.

Die Atemluft kann diskontinuierlich oder kontinuierlich, beispielsweise über eine Atemmaske und eine Leitung, der Messvorrichtung zugeführt werden. Eine Auswertevorrichtung ist beispielsweise ein Computer, insbesondere ein Laptop, auf dem der erfindungsgemäß verwendete Algorithmus in Form einer Software installiert ist. Die Auswertung ist numerisch so effizient, dass mit der Rechenkapazität eines gängigen Laptops, der vorzugsweise zur Steuerung der Messvorrichtung dient, die $^{13}CO_2$-Messdaten vorzugsweise in einigen Minuten, beispielsweise in etwa 1 Min, ausgewertet sind. Klinisch relevante Parameter stehen damit direkt nach Erstellung des Messprotokolls zur Verfügung.

Das Verfahren der vorliegenden Erfindung weist zahlreiche Vorteile auf:
Die Verwendung eines $^{13}C$-Atemgas-Tests an sich ist sehr vorteilhaft, da $^{13}C$-Atemgas-Tests nicht-invasiv, nicht radioaktiv, sicher, einfach und effektiv durchzuführen sind.

Der Test kann unmittelbar beim Patienten, beispielsweise am Bett des Patienten, durchgeführt werden und in kurzer Zeit stehen die Ergebnisse zur Verfügung. Der Patient muss nicht mehrere Stunden oder Tage auf die Ergebnisse warten.

Auch aus den einzelnen Schritten des Auswerte-Algorithmus ergeben sich einige Vorteile:
Die Darstellung der Transfer-Funktion als Polynom hat die folgenden Vorteile:

- Der Verlauf der Transfer-Funktion ist praktisch beliebig, bis auf eine Einschränkung, dass sie zum Zeitpunkt null gleich null ist.
- Es erfolgt keine Festlegung auf Bereiche, an der die Freisetzung maximal ist.

Die Regularisierung bietet folgende Vorteile:

- Mit der Wahl des Regularisierungsfaktors $\alpha$ kann die Biegsamkeit der Freisetzungskurve R(t) und damit die Anpassung der resultierenden Funktion (hier z) an die Messwerte gesteuert werden.
- Da die Polynomkoeffizienten über eine Anpassung an die Messwerte bestimmt werden, konzentrieren sich die Krümmungen auf Bereiche, in denen sie notwendig sind.

Die Marginalisierung bietet folgende Vorteile:

- der Kompromiss zwischen möglichst starrer Freisetzungskurve und möglichst guter Anpassung an die Messdaten wird automatisch gefunden. Die Kompromisslösung entspricht der Lösung, die nach statistischen Kriterien am wahrscheinlichsten ist.
- Der Stoffwechselparameter $\gamma$ und der $\alpha$-Wert sind die einzigen Parameter die über eine Anpassung bestimmt werden müssen. Die Polynomkoeffizienten werden in einem Zwischenschritt, in Abhängigkeit von $\alpha$ und $\gamma$, berechnet. Die Anzahl der Parameter, die über ein Optimierungsverfahren angepasst werden müssen, reduziert sich von etwa 27 auf 2. Da der Rechenaufwand zur Optimierung vom Quadrat der Anzahl der optimierten Parameter abhängt, reduziert sich der Rechenaufwand um etwa den Faktor 100. Dies ermöglicht eine Auswertung vor Ort, so dass medizinisch relevante Größen sofort nach Beendigung der Messungen zur Verfügung stehen.
- Der kinetische Koeffizient ($k_1$) kann parallel zum $\alpha$-Wert aus demselben Datensatz bestimmt werden. Damit ist eine getrennte Bestimmung der Menge möglich, die vom Magen-/Darm zum Ort des Stoffwechsels gelangt, und von der Rate, mit der die angekommene Menge oxidiert wird.

Die sehr allgemein gehaltene mathematische Formulierung für R(t) erlaubt es, generell jede Form von R(t), bei allerdings etwas eingeschränkter 'Biegsamkeit', darzustellen, so dass Sprünge oder abrupte Änderungen ausgeschlossen werden. Erfindungsgemäß können damit auch irreguläre $^{13}CO_2$-Freisetzungsfunktionen ausgewertet werden. Das erfindungsgemäße Verfahren kann daher gleichermaßen bei Gesunden und Kranken eingesetzt werden. Bei gesunden Personen kann eine irreguläre Freisetzung beispielsweise medikamentös induziert sein.

Benötigt werden lediglich die Menge der Dosis der verabreichten Testverbindung, die Messwerte für die

$^{13}CO_2/^{12}CO_2$ Anreicherungen und deren Zeitpunkte sowie patientenspezifische Werte, wie Gewicht, Größe, Alter des Patienten. Damit ist die Auswertung standardisierbar.

Gemäß der vorliegenden Erfindung erfolgt vorzugsweise eine automatisierte Auswertung. Dies ergibt sich aus der Tatsache dass nur unbekannte Größen für den Regularisierungskoeffizienten $\alpha$ und für den Stoffwechselparameter $\gamma$ ermittelt werden müssen, wenn die berechneten $^{13}CO_2$-Freisetzungskurven den entsprechenden Messwerten angepasst werden. Zur Abschätzung des Splitfaktors f aus dem Stoffwechselparameter $\gamma$ wird zusätzlich eine Bestimmung der Gesamt-$CO_2$-Produktion pro Zeiteinheit benötigt.

Techniken des "machine learnings" können angewendet werden, um eine intelligente Auswertesoftware zu erstellen, die eine online-Auswertung der Messdaten auf "Knopfdruck" erlaubt, ohne dass das Bedienungspersonal auf die speziellen Testdaten eingehen muss. Dies wird in den Gleichungen (13) bis (16) realisiert. Damit eröffnet sich die Möglichkeit, die Testauswertung zu standardisieren und den Test in einfacher Form durchzuführen, d.h. ein einzelner Messdatensatz kann ohne spezielle Benutzereingaben ausgewertet werden.

Die Auswertung kann mit einem üblichen Laptop durchgeführt werden, wobei in sehr kurzer Zeit, beispielsweise nach etwa 1 Min, bereits die ausgewerteten Daten zur Verfügung stehen.

Das erfindungsgemäße Testverfahren kann diskontinuierlich oder kontinuierlich durchgeführt werden.

Die Messung und Auswertung kann in kompakter Weise durchgeführt werden, ohne großen Platzbedarf, so dass beispielsweise mobile Vorrichtungen direkt an das Krankenbett gebracht oder auch in der Intensivstation eingesetzt werden können.

Die berechneten Freisetzungskurven geben Auskunft darüber, ob eine mehrphasige oder gar völlig irreguläre Entleerung vorliegt und wie schnell die Entleerung des Magens erfolgt und wann in etwa die maximale Entleerung stattfindet. Darüber hinaus können Daten zur Verstoffwechselung ermittelt werden. Es werden insbesondere bei kranken Patienten und auch bei Intensiv-Patienten zuverlässige Ergebnisse erhalten.

Anhand der mit dem erfindungsgemäßen Verfahren zur Verfügung gestellten Transfer-Funktionen R(t) und dem Stoffwechselparameter $\gamma$ bzw. Splitfaktor f kann sich der Arzt eine geeignete Behandlungsstrategie überlegen.

Die vorliegende Erfindung soll nachfolgend anhand von einem Ausführungsbeispiel im Einzelnen erläutert werden, ohne diese hierauf zu beschränken.

**Anwendungsbeispiel**

Ergebnisse eines Pilotversuches

Ein Proband führte einen erfindungsgemäßen $^{13}$C-Atemgas-Test mit Acetat als Testverbindung durch. Die Testverbindung wurde in einer Menge von 200 mg einmal morgens nüchtern, und zwei Tage später morgens nach dem Frühstück nochmals 200 mg in einer Nährlösung eingenommen.

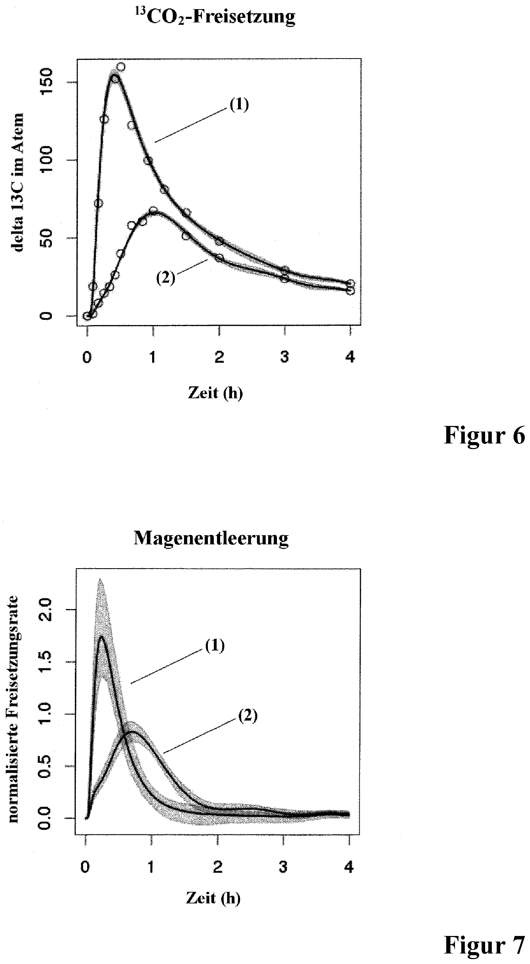

Figur 6 zeigt die ermittelten Freisetzungskurven, d.h. die gemessenen $\delta$ $^{13}$C-Messdaten, ($\delta$ $^{13}$C ist ein übliches Maß der $^{13}$C-Anreicherung, sie entspricht ungefähr: Anreicherung in Prozent *1000) aufgetragen gegen die Zeit in Stunden. Die $\delta$-Werte sind die Änderungen der relativen Konzentrationen bzw. Anreicherungsdaten, die als Differenz zu den in der Ausatemluft üblicherweise vorliegenden $^{13}CO_2$-Werten bestimmt werden. Die Kreise sind die gemessenen $\delta$ $^{13}$C-Anreicherungsdaten im Atemgas-Test gemäß der vorliegenden Erfindung. Kurve (1) zeigt die Freisetzungskurve in nüchternem Zustand, Kurve (2) zeigt die Freisetzungskurve zwei Tage später nach dem Frühstück.

In Figur 7 sind anhand der in Figur 6 gemessenen Anreicherungsdaten die optimalen Transfer-Funktionen R(t), die anhand der erfindungsgemäß entwickelten mathematischen Abschätzung errechnet wurden, dargestellt. Die durchgezogenen Linien sind entsprechende Modellwerte, berechnet nach Gleichung (1). Die für dieses Ausführungsbeispiel berechneten $\gamma$-Werte sind 270 (255; 288) für den Nüchtern-Zustand und 159 (150; 169) nach dem Frühstück. Die Werte in Klammern stellen dabei die 95%-Konfidenz-Bereiche dar. Die Gesamt-$CO_2$-Produktion wurde mit 600 mmol/Stunde abgeschätzt. Hieraus wurde der Splitfaktor mit 0,47 nüchtern und 0,28 nach dem Frühstück berechnet. In der Regel werden nüchtern etwa 30% der verabreichten markierten Acetat-Kohlenstoff Atome über die Atmung wieder freigesetzt. Der Splitfaktor liegt jeweils im normalen zu erwartenden Bereich zwischen 0,2 und 0,6.

Im gezeigten Beispielfall handelt es sich ansatzweise um eine irreguläre Freisetzungskurve. Insbesondere die Kurve (2) in Figur 7, die das Freisetzungsverhalten nach dem Frühstück wiedergibt, zeigt einen zweiten Peak bei etwa 2,5 Stunden. Dies ist in den entsprechenden Messdaten in Figur 6 nur angedeutet. Ein stärker ausgeprägter zweiter Peak oder eine 'Schulter' am Rand des Hauptpeaks in der $^{13}CO_2$-Freisetzung sollte demnach in einem zweiten Peak in R(t) reflektiert werden.

Die Flächen um die Kurven bedeuten den 95% Konfidenz-Bereich, wie bereits erläutert.

Diese Ergebnisse zeigen, dass aus $^{13}CO_2$-Anreicherungsdaten sowohl der Stoffwechselparameter $\gamma$ als auch die Transfer-Funktion R(t) mit guter Genauigkeit bestimmt werden können. Figur 7 zeigt dabei, dass auch Freisetzungskurven ausgewertet werden können, die nicht nur einen Peak zeigen.

Ein Vergleich von Figur 6 und 7 zeigt außerdem, dass die Transfer-Funktionen R(t) nach etwa 3 Stunden nahe null sind, während die Anreicherungsdaten nach 4 Stunden immer noch messbar über Null liegen. Die gesamte abgegebene Menge an $^{13}CO_2$, die Ausgangspunkt anderer Verfahren aus dem Stand der Technik ist, kann daher nicht bestimmt werden, dies ist für das erfindungsgemäße Verfahren aber auch nicht erforderlich. Die Rückrechnung auf die Transfer-Funktionen R(t) ist möglich, weil das $^{13}CO_2$-Verteilung-System, das in der rechten Hälfte von Figur 3 dargestellt ist, die Freisetzung von $^{13}CO_2$ im Atemgas zwar verzögert, diese Verzögerung aber in den Modellgleichungen erfasst wird.

Die errechneten Transfer-Funktionen R(t) haben einen engen 95% Konfidenz-Bereich, ebenso der Stoffwechselparameter $\gamma$, d.h. diese Größen können mit einer Präzision von etwa 5% des Nominal-Wertes erfasst werden. Die 5% resultieren in der Unsicherheit, die aus dem Messfehler und der limitierten Anpassung von den berechneten an die gemessenen $^{13}CO_2$-Werte entsteht.

**Patentansprüche**

1. $^{13}$C-Atemgas-Test zur Überprüfung der Magen-/Darm-Funktion und/oder der Stoffwechselfunktionen bei einem Patienten, umfassend die nachfolgenden Schritte:

   - Verabreichen einer mit $^{13}$C-markierten Verbindung an einen Patienten, durch den die $^{13}$C-markierte Verbindung zu einem $^{13}$C-markierten Metabolisierungsprodukt umgewandelt wird, wobei das $^{13}$C-markierte Metabolisierungsprodukt $^{13}CO_2$ darstellt;
   - Bestimmen der Änderung der relativen Konzentrationen des $^{13}CO_2$ in der Atemluft des Patienten über ein vorbestimmtes Zeitintervall in Form von gemessenen Anreicherungsdaten; und
   - Auswerten der erhaltenen Anreicherungsdaten anhand einer mathematischen Abschätzung der $^{13}CO_2$-Freisetzungskurve, die aufweist:

      - Formulieren von Differentialgleichungen zur Erfassung der Kinetik der $^{13}$C-markierten Verbindung im Körper des Patienten anhand bekannter kinetischer Koeffizienten;
      - Erstellen linearer Matrixgleichungen aus den Differentialgleichungen unter Berücksichtigung des zeitlichen Verlaufs des Transfers der $^{13}$C-markierten Testverbindung in die zentrale Zirkulation des Körpers des Patienten anhand einer Transfer-Funktion $R_{(t)}$ in Form eines Polynoms unter Erhalt einer Gleichung, die eine Beziehung zwischen Polynomkoeffizienten für die Transfer-Funktion R(t) und den gemessenen Anreicherungsdaten herstellt;
      - Regularisieren der erhaltenen Gleichung durch Kontrolle der Biegsamkeit der Transfer-Funktion R(t) über den Regularisierungskoeffizient $\alpha$ unter Beschränkung der Größe der Polynomkoeffizienten, wobei je kleiner die Beschränkung ist, um so größer die Biegsamkeit ist, unter Erhalt einer regularisierten Gleichung;
      - Umwandeln der regularisierten Gleichung in eine Wahrscheinlichkeit, dass ein bestimmter Datensatz in Abhängigkeit vom Regularisierungskoeffizienten a, den kinetischen Koeffizienten, einem Stoffwechselparameter $\gamma$ und den Polynomkoeffizienten gemessen wird;
      - Marginalisieren der Wahrscheinlichkeit, so dass nur eine Wahrscheinlichkeit berechnet wird, die vom Regularisierungskoeffizienten $\alpha$ und vom Stoffwechselparameter $\gamma$ abhängt, wobei die Polynomkoeffizienten vom Regularisierungskoeffizienten $\alpha$ und dem Stoffwechselparameter $\gamma$ abhängen; und
      - automatische Optimierung der Wahrscheinlichkeit unter Variieren des Regularisierungskoeffizienten $\alpha$ und des Stoffwechselparameters $\gamma$ unter Erhalt der wahrscheinlichsten Werte für $\alpha$ und $\gamma$ und damit auch der Polynomkoeffizienten; und

   - Rückrechnung der Magen-/Darm-Entleerung in Form der Transfer-Funktion R(t) aus den erhaltenen Polynomkoeffizienten und Bestimmen der Verstoffwechselung der $^{13}$C-markierten Verbindung zu $^{13}CO_2$ aus dem Stoffwechselparameter $\gamma$.

2. $^{13}$C-Atemgas-Test nach Anspruch 1, **dadurch gekennzeichnet, dass** der Test bei einem gesunden oder kranken Patienten durchgeführt wird, wobei der kranke Patient eine gestörte Magen-/Darm-Funktion und/oder gestörte Stoffwechselfunktionen hat, wobei der kranke Patient insbesondere ein Intensiv-Patient und/oder künstlich beatmeter Patient ist.

3. $^{13}$C-Atemgas-Test nach Anspruch 1 oder 2, **dadurch gekennzeichnet, dass** für das Regularisieren und Marginalisieren Methoden des ,machine learning' zugrunde gelegt werden.

4. $^{13}$C-Atemgas-Test nach einem der vorangehenden Ansprüche 1 bis 3, **dadurch gekennzeichnet, dass** aus dem

Stoffwechselparameter $\gamma$ der Splitfaktor f anhand der zusätzlich gemessene Gesamt-$CO_2$-Produktion pro Zeiteinheit des Patienten berechnet wird, wobei der Splitfaktor f den prozentualen Anteil der Menge der verabreichten Testverbindung darstellt, der vom Körper des Patienten oxidiert wird, und als Maß für die Stoffwechselfunktionen herangezogen werden kann.

**5.** $^{13}$C-Atemgas-Test nach einem der vorangehenden Ansprüche 1 bis 4, **dadurch gekennzeichnet, dass** die Anreicherungsdaten aus der Atemluft eines Patienten gemessen werden bis zumindest etwa 70% der Menge des $^{13}$C-markierten Metabolisierungsprodukts, die über Oxidation entstanden ist, abgeatmet wurden.

**6.** $^{13}$C-Atemgas-Test nach einem der vorangehenden Ansprüche 1 bis 5, **dadurch gekennzeichnet, dass** die $^{13}$C-markierte Verbindung ausgewählt wird aus einer Verbindung, die über den Stoffwechsel zu $^{13}CO_2$ abgebaut wird und die spezifisch in einem Organ abgebaut wird, wobei das Organ ausgewählt ist aus dem Magen-/Darm-Trakt, der Galle oder der Leber.

**7.** $^{13}$C-Atemgas-Test nach einem der vorangehenden Ansprüche 1 bis 6, **dadurch gekennzeichnet, dass** die $^{13}$C-markierte Verbindung in einer derartigen Menge verabreicht wird, dass ein messbares $^{13}CO_2$-Signal erzeugt wird, insbesondere mindestens 10 $\delta$ $^{13}$C beträgt.

**8.** $^{13}$C-Atemgas-Test nach einem der vorangehenden Ansprüche 1 bis 7, **dadurch gekennzeichnet, dass** die Messung über eine Dauer von 30 Minuten bis 6 Stunden durchgeführt wird und dass bevorzugt die Auswertung innerhalb von wenigen Minuten durchgeführt wird.

**9.** $^{13}$C-Atemgas-Test nach einem der vorangehenden Ansprüche 1 bis 8, **dadurch gekennzeichnet, dass** der Test kontinuierlich oder diskontinuierlich, bevorzugt diskontinuierlich, durchgeführt wird.

**10.** $^{13}$C-Atemgas-Test nach einem der vorangehenden Ansprüche 1 bis 9, **dadurch gekennzeichnet, dass** die Anreicherungsdaten unter Verwendung von IR- oder Laser-spektroskopischen Verfahren, insbesondere über die NDIR-Messtechnik (Nicht-Dispersive-Infrarot-Spektroskopie), bestimmt werden.

**11.** $^{13}$C-Atemgas-Test nach einem der vorangehenden Ansprüche 1 bis 10, **dadurch gekennzeichnet dass** die Auswertung vollständig automatisiert durchgeführt wird.

**12.** $^{13}$C-Atemgas-Test nach einem der vorangehenden Ansprüche 1 bis 11, **dadurch gekennzeichnet, dass** zusätzlich zu dem Regularisierungskoeffizienten $\alpha$ und dem Stoffwechselparameter $\gamma$ die kinetischen Koeffizienten variiert und optimiert werden, mit der Einschränkung, dass die resultierenden Werte sich im Bereich der bekannten Wahrscheinlichkeitsverteilung für einen kinetischen Koeffizient befinden,
wobei vorzugsweise so vorgegangen wird, dass die kinetischen Koeffizienten, basierend auf bereits bekannten Wertebereichen, anhand eines Bayesian-Approach entsprechend bestimmt werden.

**13.** $^{13}$C-Atemgas-Test nach einem der vorangehenden Ansprüche 1 bis 12, **dadurch gekennzeichnet, dass** wenn die Anzahl der gemessenen Anreicherungsdaten größer ist als der Grad des Polynoms, die Regularisierung weggelassen werden kann, da dann der Wert für $\alpha$ gleich Null gesetzt werden kann.

**14.** $^{13}$C-Atemgas-Test nach einem der vorangehenden Ansprüche 1 bis 13, **dadurch gekennzeichnet, dass** anstelle eines Polynoms für die Transfer-Funktion R(t) andere Funktionen verwendet werden, die nicht-linear mit der Zeit, aber linear in den Koeffizienten sind, so dass aufgrund der Linearität in den Koeffizienten eine Marginalisierung möglich ist.

**15.** Vorrichtung zur Durchführung des Verfahrens nach einem der vorangehenden Ansprüche 1 bis 14, umfassend

- eine Messvorrichtung zur Bestimmung der Änderung der relativen Konzentrationen des $^{13}$C-markierten Metabolisierungsprodukts in Form von $^{13}CO_2$ in der Atemluft des Patienten über ein vorbestimmtes Zeitintervall unter Erhalt von Anreicherungsdaten;
- Vorrichtungen zur Entnahme der Gasprobe, insbesondere eine Pumpe, Filter, Ventile und Gaswege in Form von Schläuchen aus Gummi, Kunststoff und/oder Metall, mit oder ohne Beheizung;
- Vorrichtungen für eine interne Spannungsversorgung;
- Kommunikationseinrichtungen zur Übertragung von Mess- und Steuerdaten und
- eine Auswertevorrichtung, die derart eingerichtet ist, dass sie die erhaltnen Anreicherungsdaten anhand der mathematischen Abschätzung, die in den Ansprüchen 1 bis 14 dargelegt ist, auswertet.

**Claims**

1. $^{13}$C breath gas test for testing gastrointestinal function and/or metabolic functions in a patient, comprising the following steps:

   - administering a $^{13}$C-labeled compound to a patient, by which the $^{13}$C-labeled compound is converted into a $^{13}$C-labeled metabolism product, wherein the $^{13}$C-labeled metabolism product represents $^{13}$CO$_2$;
   - determining the change in relative concentrations of $^{13}$CO$_2$ in the patient's breath over a predetermined time interval in the form of measured enrichment data; and
   - evaluating the enrichment data obtained using a mathematical estimate of the $^{13}$CO$_2$ release curve that exhibits:

      - formulating differential equations to acquire the kinetics of the $^{13}$C-labeled compound in the patient's body using known kinetic coefficients;
      - preparing linear matrix equations from the differential equations taking into account the temporal course of the transfer of the $^{13}$C-labeled test compound into the central circulation of the patient's body using a transfer function $R_{(t)}$ in the form of a polynomial to obtain an equation which establishes a relationship between polynomial coefficients for the transfer function $R_{(t)}$ and the measured enrichment data;
      - regularizing the obtained equation by checking the flexibility of the transfer function $R_{(t)}$ via the regularization coefficient $\alpha$ while limiting the size of the polynomial coefficients, wherein the smaller the constraint, the greater the flexibility, by obtaining a regularized equation;
      - converting the regularized equation into a probability that a given data set will be measured as a function of the regularization coefficient $\alpha$, the kinetic coefficients, a metabolic parameter $\gamma$ and the polynomial coefficients;
      - marginalizing the probability so that only a probability is calculated that depends on the regularization coefficient $\alpha$ and the metabolic parameter $\gamma$, wherein the polynomial coefficients depend on the regularization coefficient $\alpha$ and the metabolic parameter $\gamma$; and
      - automatically optimizing the probability by varying the regularization coefficient $\alpha$ and the metabolic parameter $\gamma$ to obtain the most probable values for $\alpha$ and $\gamma$ and thus also the polynomial coefficients; and

      - back-calculation of the gastrointestinal emptying in the form of the transfer function $R_{(t)}$ from the polynomial coefficients obtained and determination of the metabolism of the $^{13}$C-labeled compound to $^{13}$CO$_2$ from the metabolic parameter $\gamma$.

2. $^{13}$C breath gas test according to claim 1, **characterized in that** the test is carried out on a healthy or sick patient, wherein the sick patient has a disturbed gastrointestinal function and/or disturbed metabolic functions, wherein the sick patient in particular is an intensive care patient and/or artificially ventilated patient.

3. $^{13}$C breath gas test according to claim 1 or 2, **characterized in that** machine learning methods are used for regularization and marginalization.

4. $^{13}$C breath gas test according to one of the preceding claims 1 to 3, **characterized in that** the split factor f is calculated from the metabolic parameter $\gamma$ on the basis of the additionally measured total CO$_2$ production per unit time of the patient, wherein the split factor f represents the percentage proportion of the amount of the administered test compound which is oxidized by the body of the patient and can be used as a measure for the metabolic functions.

5. $^{13}$C breath gas test according to one of the preceding claims 1 to 4, **characterized in that** the enrichment data are measured from the respiratory air of a patient until at least about 70% of the amount of the $^{13}$C-labeled metabolization product produced by oxidation has been breathed out.

6. $^{13}$C breath gas test according to one of the preceding claims 1 to 5, **characterized in that** the $^{13}$C-labeled compound is selected from a compound which is metabolically degraded to $^{13}$CO$_2$ and which is specifically degraded in an organ, wherein said organ is selected from the gastrointestinal tract, gall bladder or liver.

7. $^{13}$C breath gas test according to one of the preceding claims 1 to 6, **characterized in that** the $^{13}$C-labeled compound

is administered in such an amount that a measurable $^{13}CO_2$ signal is generated, and is in particular at least 10 $\delta$ $^{13}C$.

8.  $^{13}C$ breath gas test according to one of the preceding claims 1 to 7, **characterized in that** the measurement is carried out over a period of 30 minutes to 6 hours and that preferably the evaluation is carried out within a few minutes.

9.  $^{13}C$ breath gas test according to one of the preceding claims 1 to 8, **characterized in that** the test is carried out continuously or discontinuously, preferably discontinuously.

10. $^{13}C$ breath gas test according to one of the preceding claims 1 to 9, **characterized in that** the enrichment data are determined using IR or laser spectroscopic methods, in particular NDIR (non-dispersive infrared spectroscopy) measurement technology.

11. $^{13}C$ breath gas test according to one of the preceding claims 1 to 10, **characterized in that** the evaluation is carried out in a completely automated manner.

12. $^{13}C$ breath gas test according to one of the preceding claims 1 to 11, **characterized in that**, in addition to the regularization coefficient $\alpha$ and the metabolic parameter $\gamma$, the kinetic coefficients are varied and optimized, with the restriction that the resulting values are in the range of the known probability distribution for a kinetic coefficient, preferably proceeding in such a way that the kinetic coefficients, based on already known value ranges, are determined accordingly using a Bayesian approach.

13. $^{13}C$ breath gas test according to one of the preceding claims 1 to 12, **characterized in that** if the number of enrichment data measured is greater than the degree of polynomial, the regularization can be omitted since then the value for $\alpha$ can be set equal to zero.

14. $^{13}C$ breath gas test according to one of the preceding claims 1 to 13, **characterized in that** instead of a polynomial for the transfer function $R_{(t)}$, other functions are used which are non-linear with time but linear in the coefficients, so that due to the linearity in the coefficients a marginalization is possible.

15. Device for carrying out the method according to one of the preceding claims 1 to 14, comprising

    - a measuring device for determining the change in relative concentrations of the $^{13}C$-labeled metabolization product in the form of $^{13}CO_2$ in the patient's respiratory air over a predetermined time interval by obtaining enrichment data;
    - devices for taking the gas sample, in particular a pump, filters, valves and gas passages in the form of rubber, plastic and/or metal hoses, with or without heating;
    - devices for an internal power supply;
    - communication devices for the transmission of measurement and control data; and
    - an evaluation device adapted to evaluate the obtained enrichment data based on the mathematical estimation as set forth in claims 1 to 14.

**Revendications**

1.  Test avec du gaz respiratoire marqué au $^{13}C$ pour la vérification des fonctions gastro-intestinales et/ou des fonctions métaboliques d'un patient, comprenant les étapes suivantes :

    - administration d'un composé marqué avec du $^{13}C$ au patient, lequel transforme le composé marqué au $^{13}C$ en un produit du métabolisme marqué au $^{13}C$, lequel produit du métabolisme marqué au $^{13}C$ présente du $^{13}CO_2$;
    - détermination du changement de concentration relative du $^{13}CO_2$ dans l'air respiratoire du patient sur un intervalle de temps donné sous la forme de données d'enrichissement mesurées ; et
    - analyse des données d'enrichissement obtenues à l'aide d'une estimation mathématique de la courbe de dégagement de $^{13}CO_2$, comportant :

        - la formulation d'équations différentielles pour représenter la cinétique du composé marqué au $^{13}C$ dans l'organisme du patient à l'aide de coefficients cinétiques connus ;
        - la création d'équations matricielles linéaires à partir des équations différentielles en tenant compte de l'évolution dans le temps du transfert du composé de test marqué au $^{13}C$ dans la circulation centrale du corps du patient à l'aide d'une fonction de transfert R(t) polynomiale en obtenant une équation qui crée une relation entre des coefficients polynomiaux pour la fonction de transfert R(t) et les données d'enrichissement mesurées ;

- régularisation de l'équation obtenue par le contrôle de la flexibilité de la fonction de transfert R(t) à l'aide du coefficient de régularisation $\alpha$ en limitant la grandeur des coefficients polynomiaux, la flexibilité étant d'autant plus grande que la limitation est petite, en obtenant une équation régularisée ;

- la conversion de l'équation régularisée en une probabilité qu'un certain jeu de données soit mesuré en fonction du coefficient de régularisation $\alpha$, des coefficients cinétiques, d'un paramètre métabolique $\gamma$ et des coefficients polynomiaux ;

- la marginalisation de la probabilité de façon à calculer seulement une probabilité dépendant du coefficient de régularisation $\alpha$ et du paramètre métabolique $\gamma$, les coefficients polynomiaux dépendant du coefficient de régularisation $\alpha$ et du paramètre métabolique $\gamma$, et

- l'optimisation automatique de la probabilité en faisant varier le coefficient de régularisation $\alpha$ et le paramètre métabolique $\gamma$ tout en conservant les valeurs les plus probables de $\alpha$ et $\gamma$ et ainsi des coefficients polynomiaux ;

et

- rétropolation de la vidange gastrique ou intestinale sous la forme de la fonction de transfert R(t) à partir des coefficients polynomiaux obtenus et détermination de la métabolisation du composé marqué au $^{13}C$ en $^{13}CO_2$ à partir du paramètre métabolique $\gamma$.

**2.** Test avec du gaz respiratoire marqué au $^{13}C$ selon la revendication 1, **caractérisé en ce que** le test est effectué sur un patient en bonne santé ou malade, le patient malade souffrant d'un trouble de la fonction gastrique ou intestinale et/ou d'un trouble des fonctions métaboliques, le patient malade étant en particulier un patient en soins intensifs et/ou un patient sous assistance respiratoire.

**3.** Test avec du gaz respiratoire marqué au $^{13}C$ selon la revendication 1 ou 2, **caractérisé en ce que** des méthodes d'apprentissage par la machine sont utilisées pour la régularisation et la marginalisation.

**4.** Test avec du gaz respiratoire marqué au $^{13}C$ selon l'une des revendications 1 à 3, **caractérisé en ce que** le facteur de fractionnement f est calculé à partir du paramètre métabolique $\gamma$ à l'aide de la production totale de $CO_2$ par unité de temps du patient mesurée en complément, le facteur de fractionnement f représentant le pourcentage de la quantité du composé de test administré qui est oxydé par l'organisme du patient et pouvant être utilisé comme mesure des fonctions métaboliques.

**5.** Test avec du gaz respiratoire marqué au $^{13}C$ selon l'une des revendications 1 à 4, **caractérisé en ce que** les données d'enrichissement sont mesurées dans l'air respiratoire d'un patient jusqu'à ce qu'au moins environ 70 % du produit de métabolisme marqué au $^{13}C$ formée par oxydation aient été exhalés.

**6.** Test avec du gaz respiratoire marqué au $^{13}C$ selon l'une des revendications 1 à 5, **caractérisé en ce que** le composé marqué au $^{13}C$ est choisi parmi un composé qui est décomposé par le métabolisme en $^{13}CO_2$ et qui est spécifiquement décomposé dans un organe, l'organe étant choisi parmi le tractus gastro-intestinal, la vésicule biliaire ou le foie.

**7.** Test avec du gaz respiratoire marqué au $^{13}C$ selon l'une des revendications 1 à 6, **caractérisé en ce que** le composé marqué au $^{13}C$ est administré en quantité telle qu'un signal de $^{13}CO_2$ mesurable soit produit et atteigne en particulier au moins 10 $\delta$ $^{13}C$.

**8.** Test avec du gaz respiratoire marqué au $^{13}C$ selon l'une des revendications 1 à 7, **caractérisé en ce que** la mesure est réalisée sur une durée de 30 minutes à 6 heures et **en ce que** l'interprétation est de préférence réalisée au bout de quelques minutes.

**9.** Test avec du gaz respiratoire marqué au $^{13}C$ selon l'une des revendications 1 à 8, **caractérisé en ce que** le test est effectué de façon continue ou discontinue, de préférence de façon discontinue.

**10.** Test avec du gaz respiratoire marqué au $^{13}C$ selon l'une des revendications 1 à 9, **caractérisé en ce que** les données d'enrichissement sont obtenues en utilisant des méthodes de spectroscopie IR ou laser, en particulier la technique de mesure NDIR (spectroscopie infrarouge non dispersive).

11. Test avec du gaz respiratoire marqué au $^{13}$C selon l'une des revendications 1 à 10, **caractérisé en ce que** l'analyse est exécutée de façon entièrement automatique.

12. Test avec du gaz respiratoire marqué au $^{13}$C selon l'une des revendications 1 à 11, **caractérisé en ce qu'**outre le coefficient de régularisation $\alpha$ et le paramètre métabolique $\gamma$, les coefficients cinétiques sont modifiés et optimisés, avec la restriction que les valeurs en résultant se situent dans la plage de répartition de probabilité connue pour un coefficient cinétique, la démarche étant de préférence telle que les coefficients cinétiques soient déterminés, sur la base de plages de valeurs déjà connues, à l'aide d'une approche bayésienne.

13. Test avec du gaz respiratoire marqué au $^{13}$C selon l'une des revendications 1 à 12, **caractérisé en ce que**, quand le nombre des données d'enrichissement mesurées est plus grand que le degré du polynôme, la régularisation peut être omise car la valeur de $\alpha$ peut alors être posée égale à zéro.

14. Test avec du gaz respiratoire marqué au $^{13}$C selon l'une des revendications 1 à 13, **caractérisé en ce qu'**au lieu d'un polynôme, on utilise pour la fonction de transfert R(t) d'autres fonctions non linéaires dans le temps mais linéaires au niveau des coefficients, de sorte qu'une marginalisation est possible en raison de la linéarité au niveau des coefficients.

15. Dispositif pour la mise en oeuvre du procédé selon l'une des revendications 1 à 14, comprenant

- un dispositif de mesure pour déterminer le changement des concentrations relatives du produit du métabolisme marqué au $^{13}$C sous la forme de $^{13}CO_2$ dans l'air respiratoire du patient sur un intervalle de temps prédéterminé avec obtention de données d'enrichissement ;
- des dispositif pour le prélèvement de l'échantillon de gaz, en particulier une pompe, un filtre, des robinets et des conduites de gaz formées par des tuyaux en caoutchouc, en plastique et/ou en métal, avec ou sans chauffage;
- des dispositifs pour une alimentation interne en tension ;
- des installations de communication pour la transmission de données de mesure ou de commande et
- un dispositif d'analyse configuré de façon à analyser les données d'enrichissement reçues à l'aide de l'estimation mathématique exposée dans les revendications 1 à 14.

**Figur 1**

| | |
|---|---|
| Formulierung des Modells als System von Differentialgleichungen | 1. Block |
| Lineare Matrix-Gleichung Relation zwischen Polynomkoeffizienten von R(t) und den Messwerten | 2. Block |

**Regularisierung**
Kontrolle der Biegsamkeit der Transferfunktion R(t) über den Regularisierungskoeffizienten $\alpha$

3. Block

**Marginalisierung**
Wahrscheinlichkeit, einen bestimmten Datensatz zu erhalten, Polynomkoeffizienten hängen nur vom Regularisierungskoeffizienten $\alpha$ und vom Stoffwechselparameter $\gamma$ ab

4. Block

Automatische Optimierung unter Erhalt der wahrscheinlichsten Werte für $\alpha$ und $\gamma$ und damit der Polynomkoeffizienten

5. Block

Elemente des 'machine learning'

**Figur 2**

**Figur 3**

Figur 4a

**Figur 4b**

Figur 5

## ¹³CO₂-Freisetzung

**Figur 6**

## Magenentleerung

**Figur 7**

**EP 3 220 818 B1**

**In der Beschreibung aufgeführte Patentdokumente**

- WO 03017818 A2
- US 20110313677 A1
- WO 2011026613 A2
- DE 102009039543 A1
- DE 102011007310 A1
- WO 2007107366 A1


**In der Beschreibung aufgeführte Nicht-Patentliteratur**

- **MODAK A.** Stable isotope breath tests in clinical medicine: a review. *J. Breath Res.,* 2007, vol. 1, 014003
- **D.A. SCHOELLER et al.** C abundances of nutrients and the effect of variations in C isotopic abundances of test meals formulated for CO2 breath tests. *The American Journal of Clinical Nutrition,* 1980, vol. 33, 2375-2385
- **B. BRADEN et al.** C-breath tests: Current state of the art and future directions. *Digestive and Liver Disease,* 2007, vol. 39, 795-805
- **KLEIN P.D.** Clinical applications of CO2 measurements. *Federation proceedings,* 1982, vol. 41 (10), 2698-2701
- **BRADEN B.** Methods and functions: Breath tests. *Best practice & research clinical gastroenterology,* 2009, vol. 23 (3), 337-352
- **SKURIDA G. I.** Representation of the characteristic equation of the unbranched enzymatic reaction with an arbitrary number of stages in the form of a polynomial. *Biophysics,* 1980, vol. 25, 443-446
- **VOGT J. A. ; DENZER C.** Estimation of parameters for the elimination of an orally administered test substance with unknown absorption. *Journal of Pharmacokinetics and Pharmacodynamics,* 2013, vol. 40 (2), 177-187
- **MASON J.C. ; HANDSCOMB D.C.** Chebyshev polynomials. Chapman & Hall/CRC, 2003
- **SIEHE HASTIE T ; TIBSHIRANI R ; FRIEDMAN J. H.** The Elements of Statistical Learning. Springer, 2008
- **SACCOMANI M.P. ; BONADONNA R.C. ; CAVEGGION E. ; DEFRONZO R.A. ; COBELLI C.** Bicarbonate kinetics in humans: identification and validation of a three-compartment model. *The American Journal of Physiology,* 1995, vol. 269 (1), E183-92
- **BISHOP C. M.** Pattern Recognition and Machine Learning (Information Science and Statistics). Springer, 2007
- **J.A. VOGT ; W. FABINSKI ; J. KAPPLER ; H. FISCHER ; M. GEORGIEFF.** Response surface calibration of CO2-NDIR offset values: A 'random coefficients' approach. *Chemometrics and Intelligent Laboratory Systems,* Juli 2011, vol. 107 (2), 377-383